# EUROPEAN PATENT SPECIFICATION

(11) **EP 4 196 138 B1**
(45) Date of publication and mention of the grant of the patent: **18.11.2026**
(21) Application number: 21856317.9
(22) Date of filing: 11.08.2021
(51) Int. Cl.: A61K 35/644, A61P 9/00, A61P 25/00, A61P 25/18, A61P 25/28, A61P 29/00, A61P 31/00, A61P 31/04, A61P 31/12, A61P 35/00, A61P 37/00, A61P 43/00, B01D 15/36, B01J 47/014, G01N 33/15

(54) **BEE PRODUCT EXTRACTS, METHODS OF PRODUCTION AND USES THEREOF**
BIENENPRODUKTEXTRAKTE, VERFAHREN ZUR HERSTELLUNG UND VERWENDUNGEN DAVON
EXTRAITS DE PRODUITS DES ABEILLES, PROCÉDÉS DE PRODUCTION ET UTILISATIONS ASSOCIÉS

(30) Priority: 11.08.2020 NZ 20767031
(43) Date of publication of application: 21.06.2023
(60) Divisional application: 26209234.9
(73) Proprietor: Quantum Technologies Limited, Wellington 6035 (NZ)
(72) Inventor: JOHNSON, Keryn, Wellington, 6035 (NZ)
(74) Representative: Greaves Brewster LLP
(86) International application number: PCT/NZ2021/050128
(87) International publication number: WO 2022/035326

(56) References cited:
- WO-A1-2005/120250
- AU-B2- 2013 277 858
- CN-A- 106 770 116
- CN-A- 110 973 554
- KR-A- 20030 027 482
- US-A1- 2011 076 376
- ANONYMOUS: "Products - OH BEE HAVE empowering healing", 21 January 2021 (2021-01-21), XP093147805, Retrieved from the Internet <URL:https://web.archive.org/web/20210120152916/https://www.ohbeehave.co.nz/collections/all> [retrieved on 20240404]
- LI XIAOWEI, KE YUEBIN, WANG YINGYU, WANG CHENGFEI, YE DONGYANG, HU XUE, ZHOU LAN, XIA XI: "Confirmatory Analysis of Nitroimidazoles and Hydroxy Metabolites in Honey by Dispersive-Solid Phase Extraction and Ultra-High Performance Liquid Chromatography-Tandem Mass Spectrometry", MOLECULES, vol. 23, no. 12, 18 December 2018 (2018-12-18), pages 3350, XP055908436, DOI: 10.3390/molecules23123350
- HE QIONG, YAN YIZHU, CHENG, XUE XIAOFENG, WU LIMING, CAO WEI: "Removal of Streptomycin from Honey by Cation-exchange Resin", JOURNAL OF RESIDUALS SCIENCE AND TECHNOLOGY, vol. 13, no. S1, 2016, US , pages S33 - S38, XP009543320, ISSN: 1544-8053, DOI: 10.12783/issn.1544-8053/13/2/S6
- WANG KUN; JIANG JIA; LV XINPING; ZANG SHUANG; TIAN SIZHU; ZHANG HANQI; YU AIMIN; ZHANG ZIWEI; YU YONG: "Application of solvent floatation to separation and determination of triazine herbicides in honey by high-performance liquid chromatography", ANALYTICAL AND BIOANALYTICAL CHEMISTRY, SPRINGER BERLIN HEIDELBERG, BERLIN/HEIDELBERG, vol. 410, no. 8, 25 January 2018 (2018-01-25), Berlin/Heidelberg, pages 2183 - 2192, XP036442998, ISSN: 1618-2642, DOI: 10.1007/s00216-018-0881-5

## Description

### FIELD OF THE INVENTION

The invention relates to the preparation of Bee Product Extract from Manuka honey, Bee Product Extracts obtainable by such methods and pharmaceutical compositions comprising Bee Product Extracts.

### BACKGROUND OF THE INVENTION

Honey has been used for centuries by cultures through the world for its multiple health benefits. Two of the most important health benefits of honey are its anti-bacterial and anti-inflammatory properties. Manuka honey, which is produced by bees that collect nectar from Leptospermum scoparium, a plant native to New Zealand and Southern Australia, has been identified as being a variety of honey that exhibits particularly effective anti-bacterial and anti-inflammatory properties.

Recently, it was discovered that the chemical, methylglyoxal (MGO/2-oxopropanal), is a major component of the anti-bacterial activity of manuka honey. Manuka honey samples that contain greater concentrations of MGO have a higher amount of anti-bacterial activity as compared to honey samples with lower concentrations of MGO. MGO is believed to confer antibacterial properties on honey because MGO is a highly chemically reactive compound, and MGO can readily react with cellular molecules. The chemical reactions between MGO and cellular molecules in bacteria damage molecules that are important for bacterial viability, and thereby MGO functions as an antibacterial agent. The presence of high levels of MGO in the honey is a feature that distinguishes manuka honey from other varieties of honey. While most varieties of honey exhibit some anti-bacterial activity, the anti-bacterial activity in most varieties of honey is primarily a result of the presence of hydrogen peroxide in the honey. Manuka honey, in contrast, exhibits anti-bacterial activity primarily because of the presence of MGO in the honey.

The antimicrobial properties of Manuka honey are attributed to a non peroxide activity [NPA] also known as UMF, as it has been shown that MGO reacts with and inhibits glucose oxidase [1], which prevents the generation of hydrogen peroxide in Manuka honey [1]. It is also known that blending Manuka honey with peroxide producing honey results in the loss of non peroxide antimicrobial activity in the Manuka honey and this practice is discouraged.

In light of this information, the credibility of the role MGO by itself as the unique Manuka factor (UMF) is brought into question. Previously, others have shown a correlation between MGO content and antimicrobial activity supporting this current hypothesis. The clinical use of MGO to treat infection through oral use is therefore questionable and clinical trials have not been successful, again indicating the ineffectual properties of MGO by itself as the active ingredient in Manuka honey.

Despite this the industry still appears to value Manuka honey based on its MGO content. The aging of Manuka honey and the conversion of dihydroxyacetone (DHA) to MGO has been extensively modelled. The dehydrating properties of Manuka honey, due to the low water content, means that DHA is mostly in a dimer form and not able to react to produce MGO. The dimer monomer equilibrium being rate limiting in the generation of MGO. MGO may also be polymeric and cyclic in structure. It is assumed that DHA from nectar is converted in Manuka honey to MGO.

The hydrogen peroxide can react with MGO to generate pyruvate, which can be used as a source of energy by cells. The lack of hydrogen peroxide in Manuka honey means that an alternative anti-microbial system must be present in Manuka honey. A considerable amount of effort has gone into trying to discover the bioactive responsible for the NPA activity. Many different single compounds have been identified but the concentration of these compounds in the honey is too low to account for the anti-microbial activity observed. The composition of Manuka honey has been well characterized using a wide range of techniques and specific compounds must be present in order to claim that the product is authentic Manuka honey [2].

The lack of knowledge as to the origin of MGO and DHA within Manuka honey is widespread in the industry and despite repeated attempts to increase MGO content naturally it appears that only time and temperature are the main tools the industry can use to enhance MGO content. MGO has been demonstrated to react with proteins and its reaction with glucose oxidase is associated with enzyme inhibition, which stops hydrogen peroxide production within Manuka honey and therefore the anti-microbial activity associated with Manuka honey is based on NPA or a non-peroxide activity. This has been directed towards MGO and its correlation provided the evidence for its association with antimicrobial activity.

A number of patents describe methods to recover the UMF factor from Manuka honey and the isolated active fraction has been proposed to be used in a number of different applications including anti-viral respiratory against influenza as well as its anti-microbial and anti-inflammatory activities. International patent publication number WO 2005/120250 is directed to UMF fortified honey and a method for producing UMF-containing segments of honey. Korean patent application number KR 2003-0027482 relates to a cosmetic skin-protecting composition containing manuka honey as the active ingredient.

Previously the generation of hydrogen peroxide by glucose oxidase present in honey has been attributed as the anti-microbial component present in honey. However, recent evidence has attributed the anti-microbial properties of honey to the generation of Hydroxyl radicals produced from Fenton chemistry, the reaction between reduced iron and copper with hydrogen peroxide in the honey [3].

Others have identified other antimicrobial active ingredients associated with phenolics and minerals and other small molecules in normal hydrogen peroxide producing honeys. The identification of Hydroxyl radical formation from the hydrogen peroxide, has been suggested to be the active ingredient for bactericidal activity against MRSA and VRE bacterial strains. However, there is no evidence of hydrogen peroxide formation in Manuka honey, suggesting that this mechanism does not apply with Manuka honey and its anti-bacterial activity.

The lack of hydrogen peroxide in Manuka honey means that an alternative anti-microbial system must be present in Manuka honey. A considerable amount of effort has gone into trying to discover the bioactive responsible for the NPA activity. Many different single compounds have been identified but the concentration of these compounds in the honey is too low to account for the anti-microbial activity observed. The composition of Manuka honey has been well characterized using a wide range of techniques and specific compounds must be present in order to claim that the product is authentic Manuka honey [2]. The high iron, and phenolic contents as well as its thixotropic properties makes Manuka honey difficult to work with and imparts some of the unique properties on this honey as well as the high reactive dicarbonyl species MGO and other small molecules present in the honey. MGO reactivity has been associated with radical production [4]. The rapid chemistry occurring when honey is dissolved in water means that a range of new compounds are produced rapidly and this ever changing chemical makeup makes the analysis of Manuka honey very complicated. However, honey has to be diluted to be analyzed.

A 55-75 kDa fraction has been purified by gel filtration that has anti-inflammatory activity and a fraction having a MW less than 10 kDa has been prepared by UF containing anti-inflammatory activity [5]. The presence of phenolics in Manuka honey has also been identified as having phenolics that have known anti-microbial properties but the concentration of these in the honey is too low to account for the non peroxide antimicrobial activity observed in Manuka honey. Despite the compelling story surrounding MGO in Manuka honey as the active ingredient, MGO has received attention in other medical fields related to metabolic disease and diabetes where MGO gets bad press and is associated with advanced glycation end products as the dicarbonyl MGO compound is highly reactive toward lysine, arginine and cysteine amino acids in proteins forming both reversible and irreversible reaction products. Therefore, the functional role MGO plays in health and well-being from Manuka honey has bought into question by contrasting scientific evidence. In addition, the presence of the glyoxylase pathway in cells enables the transformation of MGO into lactate which is involved in cellular energy metabolism.

The high iron, and phenolic contents as well as its thixotropic properties makes Manuka honey difficult to work with and imparts some of the unique properties on this honey as well as the high reactive dicarbonyl species MGO and other small molecules present in the honey. MGO reactivity has been associated with radical production [4]. The rapid chemistry occurring when honey is dissolved in water means that a range of new compounds are produced rapidly and this ever changing chemical makeup makes the analysis of Manuka honey very complicated. However, honey has to be diluted to be analyzed. Anti-inflammatory properties of Manuka honey have been identified and attributed to the major royal jelly proteins present in the honey and their MGO modifications. The majority of the beneficial properties attributed to Manuka honey have also been attributed to royal jelly, phenolics and other compounds present within the honey. Compounds that have been identified to both stimulate the immune system and inhibit it, have been identified by various groups.

A study suggests that royal jelly has anti-inflammatory actions brought about by an inhibition of the proinflammatory cytokine production, such as TNF-C., IL-6 and IL-1, by activated macrophages [6]. The study further suggested that the active fractions or components from the royal jelly were between 5 kDa and 30 kDa molecular weights. This study perhaps explains why most honeys have a weak anti inflammatory effect because of the royal jelly proteins that occur in honey.

While multiple mechanisms of action of the anti-bacterial activity of manuka honey are understood, the mechanisms whereby manuka honey functions as an anti-inflammatory agent have remained unknown. There is a need to develop anti-inflammatory agents based on honey, because many anti-inflammatory agents currently available have major drawbacks to their use. For example, COX-2 inhibitors, a form of non-steroidal anti-inflammatory drug (NSAID), may increase the risk of heart attack and stroke in patients, and aspirin may increase the risk of gastrointestinal bleeding. Additionally, corticosteroids are reported to inhibit the growth of epithelial cells and NSAID's are reported as being cytotoxic so both of these classes of anti-inflammatory agents are unsuitable for use in wound care. Anti-inflammatory agents derived from honey may have fewer toxic side effects in one or more areas than drugs currently available, and may also offer different possible uses than anti-inflammatory drugs currently available.

The development of antibacterial resistance due to widespread usage of antibiotics has resulted in a humanitarian crisis whereby multiple drug resistance is now evidenced throughout the world. The failure of the last line of defense has resulted in a great need for the development of antimicrobials based on the natural potency present in honey. As manuka honey has non peroxide based antimicrobial actiivity the identification of the mode of action is therefore highly important to overcome the issues around antibiotic resistance.

Kanuka and manuka honey have shown antiviral activity. The current global pandemic is therefore highlighted the need for safe and effective antivirals to prevent the widespread misery that is currently occuring due to SARS-CoV-2.

The nootropic benefits of manuka honey have been suggested due to the presence of phenolics present within the honey and as manuka honey contains a high level of phenolic compounds it is therefore implied that manuka honey will have health promoting benefits to do with mind biochemistry due to these phenolics. Recently a paper has outlined the range of phenolics associated with the nootropic benefits some of the phenolics mentioned in the paper are known to be present in manuka honey.

The anti-cancer potential of manuka honey has not been explored previously. However, though the full mechanism is yet to be fully understood, studies have shown that honey has anticancer effect through its interference with multiple cell-signaling pathways, such as inducing apoptosis, anti-proliferative, anti-inflammatory, and anti-mutagenic pathways. Honey modulates the body immune system. All of these benefits contribute together to enable an anti-cancer phenotype of cells exposed to low concentrations of honey.

### Proteins in honey

The presence of colloidal material in honey was identified back in 1930's by Paine et al. The colloid material was present in various honeys at a concentration ranging from 0.1% to 1%. The honeys with higher colloid content were darker in colour and thixotropic in nature which suggests that the viscosity of the honey is influenced by the colloidal (protein material within the honey). The colloidal material has also been shown to influence colour, taste, and some biological properties of honey. It was shown by Lothrop and Paine (1931) that the colloidal material upon heating decomposed and increased the colour of the honey. Dark Buckwheat honey had lower economic value due to its strong flavour and dark colour. Several methods were used to remove the colloidal from the honey to lighten its colour in order to produce a more consumer friendly product. However, the inventor and others have found that the colloidal material which is responsible for the high viscosity of honeys such as the BuckWheat and Manuka honey and Bush honey in Australia is also responsible for a considerable amount of the biological properties of the honey due to the royal jelly proteins. The composition of the colloidal material in Manuka honey is complex in nature and consists of phenolics, proteins, lipids, sugars, small molecules and minerals. The composition of the colloidal MRJP1 complex is enriched for these components which are present in a complex structure.

In general, research on royal jelly proteins has been conducted on proteins extracted from royal jelly, rather than from honey. Methods to purify royal jelly proteins have been described, including a protocol for purifying royal jelly proteins from fresh royal jelly (RJ) samples collected from Apis mellifera and Apis cerana colonies, using a combination of centrifugation, ultrafiltration, cation exchange and column chromatography [7].

Recently the antimicrobial properties have been shown to be associated with the colloidal proteins and much work has been performed over 80 odd years on the understanding of the properties of the colloids in honey starting back in 1930's and more recently the x-ray crystal structure of MRJP1 apisimin complex [8]. The recent work has identified the complex processes that occur during honey formation and how the composition of the honey affects the size and charge distribution of the proteins in honey. The complexity of the structures and the role in energy activity with respect to glucose oxidase has also been examined leading to conclusions regarding the potential usefulness of such proteins in drug delivery and owing to the natural GRAS nature of honey the product is expected to receive GRAS status and therefore be of benefit in the growing concern in human disease.

The purification of such proteins has been outlined using biochemical methods based on chromatography of a wide range of approaches, however, despite extensive efforts no prior mass protein based purification methods have been conducted at industrial scale for product manufacture because the addition of water to honey activates glucose oxidase and leads to the formation of hydrogen peroxide which then reacts with components in the hydrogen to generate a wide range of compounds. The complexity of this process and its speed has out smarted scientific investigation to date. Therefore, people have stuck to producing Manuka honey in various formulations to provide therapeutic benefit for a range of conditions but this is still typically honey of some form or another and bound to other ingredients so that in can be more useful in its application to either wounds or in cold sore treatments. No commercial methods therefore exist to extract large amounts of the active ingredient from Manuka honey as the active ingredient has been identified as MGO and MGO is commercially available synthetically. It signifies that adulteration of honey is possible for the purpose of gaining economic benefit, as the money value of Manuka is based on MGO content, at least it is in the industry. The authenticity of Manuka honey has been changed recently through the introduction of a standard. This standard measures a range of components to ensure that they are of sufficient content to allow it to be marketed as Manuka honey.

The composition of the colloidal material in Manuka honey is complex in nature and consists of phenolics, proteins, lipids, sugars, small molecules and minerals. The composition of the colloidal MRJP1 complex is enriched for these components which are present in a complex structure. The complex was isolated out of royal jelly [8].

This is an example of the scientific method used to produce a high purity MRJP protein and the complexity, number of steps, time and costs involved are considerable. This cannot therefore be used as a method for industrial production of MRJP proteins from Manuka honey and clearly teaches complexity and multiple steps to produce a highly refined product using standard methods taught in protein purification.

The protein content of honey ranges from 0.1 to approximately 1% [9]. The proteins in honey come predominately from the bee and include the major royal jelly proteins, apisimin, defensin 1 and a range of others that have been identified by MS analysis [10]. Recently, the X-ray crystal structure of MRJP1 complex was determined and the presence of apisimin and 24 methylene cholesterol identified [11]. There are 9 known MRJP proteins. Protein was originally identified in honey back in the 1930's. The proteins were often removed with Bentone because they imparted properties to the honey that made it less appealing to the consumer. The functional health benefits of royal jelly proteins have been known based on their oral use and for skin health for a considerable amount of time. Royal jelly is used as a nutraceutical and health food supplement but has limited clinical substantiation.

Major royal jelly proteins (MRJPs) are a family of proteins secreted by the honey bee. The family consists of nine proteins, of which MRJP1 (also called royalactin), MRJP2, MRJP3, MRJP4, and MRJP5 are present in the royal jelly secreted by worker bees. MRJP1 is the most abundant, and largest in volume. The five proteins constitute 82-90% of the total proteins in a royal jelly. Royal jelly is a nutrient-rich mixture of vitamins, sugars, fats, proteins and enzymes. It is used for feeding the larvae. Royal jelly has been used in traditional medicine since ancient times, and the MRJPs are shown to be the main medicinal components. They are synthesized by a family of nine genes (*mrjp* genes), which are in turn members of the *yellow* family of genes such as in the fruit fly (*Drosophila*) and bacteria. They are attributed to be involved in differential development of queen larva and worker larvae, thus establishing division of labour in the bee colony.

It is known from analysis that honey contains a small amount of protein, typically less than 1% based on food label claims. The composition of honey is such that 80% is sugar, around 18% water and the remaining 2% consists of amino acids, peptides, proteins, phenolics, low molecular weight acids, fats and lipids and minerals. The minor components therefore comprise of a small portion of the honey and have been demonstrated as being of little importance other than the recent discovery of the monofloral manuka honey and MGO and its antibacterial and properties.

So no individual bioactive from Manuka honey has reached the consumer market as a single compound. Due to a lack of understanding of how Manuka honey generates its bioactivity the science has taken a back seat and been replaced by marketing. No commercial products are on the market because the small amount of active isolated from Manuka honey 0.1% remains uneconomic and unstable due to the nature of the chemical reactions that occur to produce the active ingredient. The raw material Manuka honey is aged and fetches a premium in the market based on its MGO content, around $60 / kg, therefore, as a starting material for an industrial process it is expensive and therefore taking 0.1% to 1% out of the product is deemed uneconomic.

MRJPs (as whole royal jelly) are used in the pharmaceutical and cosmetic fields, and are commercialized as an over-the-counter food supplements. They have antimicrobial activities against bacteria, fungi, and viruses. They also show an ability to lower blood pressure, fats in the blood (hypercholesterolemia), stop tumour growth *in vitro,* and anti-inflammation.[12]

Royal jelly has been associated with allergic reactions such as contact dermatitis, acute asthma, and anaphylaxis, which can lead to death. In a clinical diagnosis, MRJP1 and MRJP2 are found to be the main allergens.[13] They induce IgE-mediated hypersensitivity reactions thereby causing type 1 hypersensitivity.[13, 14]

There is a need for the development of new actives with anti inflammatory, nootropic, wound healing, burn healing, fertility, pain relief, anti-cancer, anti-viral, anti-bacterial, anti-fungal, or anti-diabetic activities or actives that reduce heart disease by lowering blood pressure, reduce asthma, COPD, emphysema, and/or mental health diseases of the mind.

Despite the identification of active compound and fractions of manuka honey containing the active fraction produced by gel filtration chromatography, no large scale production of a highly refined Manuka honey derived active has been developed. No commercial products are on the market because the small amount of active isolated from Manuka honey (0.1% of the honey) remains uneconomic and unstable due to the nature of the chemical reactions that occur to produce the active ingredient.

The current state of the art in protein purification using chromatography uses columns. The art of protein chromatography also teaches the use of salt based elution methods and down stream processing using ultrafiltration methods including nanofiltration. The traditional chromatography methods used and taught in the art of protein chromatography are unable to be used to purify MRJPs from manuka honey at an industrial scale due to the low concentration of proteins present in the honey.

### SUMMARY OF THE INVENTION

The present invention relates to methods of processing bee products to form a bee product extract, compositions using that extract and methods and uses of the extract or composition. Particular aspects and embodiments of the invention are summarized as follows. The invention is defined in the claims.

The present invention provides a batch method for processing manuka honey to form a Bee Product Extract, the method comprising:
a) Combining the honey with water and a cation exchange resin comprising a sulphur propyl or p-toluenesulfonyl ligand and a cellulose or agarose matrix;
b) Adjusting the pH of the mixture to a pH in the range of 2.5 - 3.0;
c) Filtering the mixture to form a solid, and optionally drying the solid;
d) Mixing the solid with water;
e) Adjusting the pH of the mixture to between 8.0 and 10.5;
f) Filtering the mixture and recovering the filtrate; and
g) Optionally, repeating steps b) to f).

The cation exchange resin may be an SP polymeric material in a solid form, preferably an agarose-based SP resin such as SP Big Beads (Invitrogen), or SP cellulose of a sufficient particle size to enable filtration and pressure based methods to recover the filtrate from the solid.

The filtration steps c) and f) may be performed by a cloth filter, filter press or RVF filtration, preferably by filter press.

The solid obtained in step c) may be washed with water at a low pH (such as the pH of step b)) to decrease the sugar content and retain the protein bound on to the resin.

The solid obtained in step c) may be filtered and pressed without washing, to remove as much sugar solution as possible before step d).

The mixture may be maintained at step c) for a long contact time, e.g. hours, days, weeks or months, to allow protein binding and/or to allow for germination of pollen present in the honey.

In the method of the invention, the resin may be separated from the liquid phase by centrifugation.

In the method of the invention the cation exchange medium may be an SP cellulose HG2 resin.

The invention also provides a Bee Product Extract obtained or obtainable by the method described and claimed herein.

The invention also provides a pharmaceutical composition comprising a Bee Product Extract obtained or obtainable by the method described and claimed herein.

The present invention also provides a pharmaceutical composition, where the composition is formulated as a topical or sublingual preparation.

### DESCRIPTION OF THE FIGURES

Figure 1 shows reaction of Nitrotetrazolium blue (NTB) with Bee Product Extract of Example 1 under a microscope exposed to UVA light.
Figure 2 shows the UV/Visible absorbance spectra of Bee Product Extract of Example 1.
Figure 3 shows SDS PAGE and coomassie staining of various bee product compositions.
Figure 4 shows the phenolic-MGO-apisimin covalent complex to the N-terminal lysine amino acid of apisimin produced in the Bee Product Extract of the invention.
Figure 5 shows particle size distribution for the Bee Product Extract of Example 1 measured by qNano analysis.
Figure 6 shows particle size distribution for the Bee Product Extract of Example 1 measured by NanoSight analysis.
Figure 7 shows the MALDI TOF mass spectrum of Bee Product Extract of Example 1.
Figure 8 shows the MALDI TOF tandem mass spectrum (MSMS) Bee Product Extract of Example 1.
Figure 9 shows the SCX HPLC elution profile of Bee Product Extract of Example 1.
Figure 10 shows the FTIR spectrum of Bee Product Extract of Example 3 brown floc.
Figure 11 shows the MALDI TOF tandem mass spectrum (MSMS) Bee Product Extract of Example 3.
Figure 12 shows SCX HPLC elution profile of Bee Product Extract of Example 3 white floc.
Figure 13 shows SCX HPLC elution profile of Bee Product Extract of Example 3, clarified solution.
Figure 14 shows a reaction scheme for Photo-Fenton chemistry breakdown of aromatic rings.
Figure 15 shows the absorbance at 590 nm of superoxide production rates for Bee Product Extract after UV light exposure.
Figure 16 shows the change in absorbance at 590 nm in response to UVA exposure, for successive dilutions of the Bee Product Extract of Example 3, white floc.
Figure 17 shows generation of bubbles in a solution of MGO 40% + Fe(ii)SO4 + H2O2 2% and phenyllactate.
Figure 18 shows generation of bubbles in Manuka honey when mixed with hydrogen peroxide.
Figure 19 shows NTB activity of manuka honey, and clover honey.
Figure 20 shows HDAC inhibition by MGO and Bee Product Extract of Example 1.

### DETAILED DESCRIPTION

The foregoing brief summary broadly describes the features and technical advantages of certain embodiments of the present invention. Further technical advantages will be described in the detailed description of the invention and Examples that follows. Novel features which are believed to be characteristic of the invention will be better understood from the detailed description of the invention when considered in connection with any accompanying figures and examples. The figures and examples provided herein are intended to help illustrate the invention or assist with developing an understanding of the invention.

The present invention relates to methods of preparing extracts from a bee product (e.g. honey), extracts prepared by those methods and to uses of those extracts. Without wanting to be bound by a theory it is thought that the extraction methods and extracts provided herein result in improved health promoting and/or therapeutic effects as elucidated, while overcoming various disadvantages. At least the invention provides the public with a useful choice.

The invention outlines the process to produce a Bee Product Extract, methods to analyze the Bee Product Extract and formulations that provide a delivery of health-promoting properties from the formulation produced. In particular the invention outlines the production of a Bee Product Extract from Manuka honey that contains proteins, phenolics and minerals, but is isolated from the majority of the sugar.

### Extraction methods

A range of methods have been used to purify and characterize major royal jelly proteins. There are a number of difficulties associated with the industrial scale purification of royal jelly proteins including collecting the protein from the queen cells from the hive. Purification from honey makes little economic sense as the concentration of protein from Manuka honey at $60 / kg for the raw material and at 0.1-1% protein content means selling a royal jelly nutraceutical for the oral market could not compete economically with comb sourced royal jelly. Therefore, Manuka honey sourced Bee Product Extract has not been seen as a viable commercial opportunity.

Removal of proteins from honey has been used to improve the aesthetic and consumer pleasing properties of honey. Manuka honey is very expensive and receives a premium in the market because of the research backing the benefits of this product. The proteins in honey have been shown to form colloidal particles in the micron size but upon dilution in water their size decreases into nanoparticles. This structural rearrangement can be attributed to changes in polarity, and changes in aggregation also occur due to pH. The presence of histidine residues on MRJP1 means that under acidic conditions the His residues are protonated giving a positive charge allowing electrostatic interactions to occur and aggregation. The dissolving of honey in water activates glucose oxidase allowing hydrogen peroxide formation which generates Hydroxyl radicals from reduced iron and copper present in the honey. The radical reactions generate a wide range of chemicals and break and create a number of bonds. These uncontrolled radical cascades are considered detrimental by the supplement industry. The widespread use of antioxidants are currently marketed with health giving properties.

As Manuka honey contains MGO, which reacts with and inhibits glucose oxidase, hydrogen peroxide is not produced and therefore the generation of Hydroxyl radicals are not formed as a consequence the proteins are not broken down and the system is relatively stable. The addition of water increases the reactivity of methylgloxal towards Arg, Lys and Cys residues which generates radicals. The degree and complexity of the reactions means little if any control over the composition of the material occurs. Therefore batch to batch variability is apparent. Speed of processing is therefore of considerable importance and the separation of glucose from the royal jelly proteins enables a stabilized product if any glucose oxidase activity remains in the honey
Methods to reduce the damage of the proteins includes preventing photo-reduction of the minerals coordinated to the phenolics. Other methods to prevent glucose oxidase activity include heating of the honey but this would accelerate radical rates of reaction increasing the rate of damage to the protein and causing further loss of functional control of the purification process. The inhibition of glucose oxidase by MGO therefore provides an opportunity to purify the proteins without too much chemistry occurring.

The large scale dissolving of Manuka honey takes considerable time, which allows a range of uncontrolled chemistry to occur. The pH of Manuka honey is around 4 to 5. When dissolved in water at this pH the histidine residues are positive and therefore the protein aggregates. These large protein aggregates are removed by filtration prior to column application under standard protein purification methods. When applied directly to a column the aggregates accumulate on the top of the resin bed and are too large to interact with the ion exchange resin therefore preventing effective purification. The particles are therefore unable to be eluted effectively.

Elution with salt increases the strength of hydrophobic interaction and the lipid like protein apisimin increases the binding strength to the resin under such circumstances. The use of pH is an alternative elution strategy but due to the deprotonation of the histidine residues the aggregates fall apart and elution of the nanoparticles occur over a large volume meaning nano-filtration was needed to concentrate the eluted protein. Ultrafiltration based methods suffer from speed of separation of the sugar from the proteins, providing ample opportunity for radical chemistry to occur and protein peptide bond cleavage resulting in a range of peptides generated as well as further MGO modification of proteins and peptides. This results in loss of manufacture control and product variability.

Taking all of these complex considerations into account it was surprising that a simple batch purification method was able to be devised to allow the rapid recovery of a functional extract from Manuka honey. This simple novel approach has been overlooked as a means to recover a therapeutically functional extract from Manuka honey, for use, for example in a topical spray formulation.

Surprisingly, the Bee Product Extract of the invention provides a topically bioavailable form of bee product extract as a spray which can provide therapeutic benefit.

Even more surprisingly, all of the health benefits of Manuka honey can be attributed to the components present in the Bee Product Extract that perform photo-fenton chemistry due to the mono-atomic minerals coordinated to the Pi electrons of the aromatic rings in the phenolics, generating reduced iron which then reacts with hydrogen peroxide produced in the body, generating Hydroxyl radicals as discussed further below. Retaining a small amount of sugar in the eluted product provides an opportunity to generate hydrogen peroxide and Hydroxyl radicals therefore changing the properties of the product as well as making the product sterile and suitable for wound care.

The optimal reaction conditions to promote Fenton chemistry occurs at around pH 4.5, which is the pH of Manuka honey. The elution of the protein off the resin at pH values above pH 6.5 as outlined by this method assist in stabilization of the reduced iron bound to the phenolic integrated into the royal jelly protein complex. Due to the size and optical properties of the royal jelly nanoparticles optical measurements are not very effective at quantifying the number of particles per mL therefore nanoparticle analysis methods are more appropriate
The Bee Product Extract of the invention is the first recovered from Manuka honey using non column based recovery methods.

Protein purification at industrial scale is an expensive process and the expectation of using such a method to purify the small amount of protein present in Manuka honey would further add to the costs of the product and therefore making the product uneconomic at scale. However, these issues have been overcome due to the novel discovery of the physics of monoatomics and the energy generated under these conditions as well as identification of high value market opportunities in brain health application, pain relief and in regenerative medicine. Thus the inventor has devised an industrial method that successfully purifies Bee Product Extract and formulates these into a topical spray with therapeutic benefit in a range of human diseases. As used herein, the term "monoatomics" refers to free metal ions such as iron and copper, which may be coordinated to pi electrons of an aromatic ring.

The recent analysis of MRJPs from royal jelly recovered from the honey comb in queen brood cells demonstrated that the protein MRJP1 forms a complex with apisimin and that this complex occurs in a particular ratio. The MRJP1 oligomer contains 24-methylenecholesterol which has known biological functions. The purification of highly refined MRJP1-apisimin complex was performed using standard chromatography methods from Bee Product Extract in the literature. Anion exchange chromatography is used to purify Bee Product Extract because the Bee Product Extract under acidic conditions bind to each other and form a large insoluble aggregate complexes that cannot bind to cation exchange resins because the complex is too large for the porosity of the resin. Therefore, attempts to use cation exchange chromatography to purify Bee Product Extract has resulted in the accumulation of the large insoluble protein complexes stuck on the top of the resin in the column making this approach ineffective in recovery and purification of Bee Product Extract.

Therefore, it was both surprising and unexpected that the recovery of Bee Product Extract from manuka honey could be performed using cation exchange resin. This finding was unexpected.

It was unexpectedly found that the presence of phenolic compounds and minerals allowed pi stacking interactions to occur with the sulphur propyl or p-toluenesulfonyl residue of the ion exchange resin. Therefore it is postulated that separation was not specifically due to ion exchange, but due to a mixed mode type interaction including hydrophobic pi electron stacking. This was demonstrated by the addition of salt which failed to elute the proteins from the resin, resulting in resin fouling and discolouration, therefore reducing the resins life expectancy and its ability to be reused. However, it is discovered that recovery of the complexes bound to the resin could be achieved by adjustment of the pH to basic conditions therefore enabling separation.

As the complexes present interact with light via photo-reduction of iron, and the presence of active glucose oxidase that becomes active when water is added to the manuka honey, the generation of hydrogen peroxide occurs, along with the generation of Hydroxyl radicals. The radical-scavenging properties of the phenolics enable the capture of the radicals which then react with the proteins to form covalent complexes. These complexes are allowed to react to generate a complex array of naturally occurring compounds that produce a wide range of biological activities that are of benefit of human health. The complex array of reactions is also seen when MALDI TOF MS analysis is performed on the Bee Product Extract as the MALDI TOF laser has been shown to promote the light reduction of iron and the photo-fenton chemistry processes that are responsible for the diversity of compounds present within the product produced from manuka honey.

The use of the light in photo-reduction of iron and the production of hydrogen peroxide by glucose oxidase present in the manuka honey when water is added provides a solid phase chemical synthesis process of incorporation of phenolic-mineral complexes with Bee Product Extract in this extraction process. The modification of lysines and the formation of CEL motifs provides the HDAC 1 inhibitory activity of manuka honey isolated Bee Product Extract. This modification of lysine also reduces the potential for allergies as the epitopes within the royal jelly protein families responsible for allergies contain lysine residues. The natural presence of high concentrations of MGO in manuka honey results in lysine modification and reduction of allergenic properties of the Bee Product Extract.

Typically protein purification is performed at low temperatures and quickly to ensure maximal recovery of intact proteins. The presence of proteases are typically inhibited to stop protein modification. Therefore the discovery that allowing long incubation periods for chemical induced solid phase synthesis on the resin in a batch reactor approach followed by removal of the sugar by filter press and a pH adjustment to recover the royal jelly protein complexes off the cation exchanger produced an bioactive extract from manuka honey that has shown a wide range of therapeutic benefits in preclinical and clinical investigations.

The solid phase chemical synthesis approach is therefore considerably different from what is described in the current art around the production of royal jelly. There are no products in the commercial market that make royal jelly or honey proteins in a formulation as a topical spray, that are produced from honey, or produced from manuka honey. The reason for this is quite obvious at $60 / kg and recovery of less than 1% of the product means that the 10g recoverable at the expense of large scale ion exchange chromatography would be inconceivable considering the cost of the starting material.

The methods provided herein mean the product acts as its own functional preservative due to the generation of Hydroxyl radicals. The continuous production of radicals would conventionally be considered problematic given the scientific literature outlining the damaging effects of Hydroxyl radicals on biological molecules. The art teaches that DNA is damaged, proteins are damaged and lipids and carbohydrates are all damaged by such a powerful oxidant. The approach of enabling photo-Fenton chemistry discussed further below has previously been seen as a dead end in potential development and commercialization of such a product. In fact, the entire New Zealand manuka honey industry is built off the back of MGO being the active ingredient and MGO content providing the antimicrobial properties for manuka honey. Therefore the approach outlined in this application clearly sits outside of the current thinking in the scientific community and brings into question the very foundations of the global health industries antioxidant health promoting story.

### Therapeutic Mechanism

The clinical evidence for MGO as the therapeutically beneficial molecule in manuka honey is brought into question when the current evidence is brought to light. 1) MGO is broken down by cells through the glyoxylase pathway into lactate. 2) MGO has been associated with AGE or RAGE activation in diabetes and other cardiovascular disease. Therefore the health benefits of MGO and its antibacterial properties are also considered to be questionable by the inventor. MGO by itself does not appear to have sufficient activity to induce the MIC levels, due to the concentration of MGO in manuka honey. Yes there is a correlation but it is only a correlation.

Previously, others have outlined that peroxide producing honey is able to generate Hydroxyl radicals and it is the Hydroxyl radical and not hydrogen peroxide responsible for the bactericidal activity. However, this mechanism has been discounted as a mechanism for non-peroxide honeys.

Without wanting to be bound by a theory, the present invention outlines a photo-Fenton chemistry mechanism based on a number of the components present in manuka honey that can generate Hydroxyl radicals leading to the identification of the mode of action. The short lived high energy radical provides the ability to convert the microorganism into CO2 and water given sufficient time and hydrogen peroxide. Furthermore, the mode of action in generating high energy short lived Hydroxyl radicals has a role in the stimulation of cellular breakdown into CO2 and water and the process of cellular death and regeneration.

The presence of CO2 release can be observed through the formation of bubbles when hydrogen peroxide is added to manuka honey under a microscope slide and the rate of bubble formation corresponds to the amount of photo-fenton chemistry. The rate of reaction can be modified by changing what wavelengths of light are used to observe the manuka honey hydrogen peroxide mixture where blue light and UVA are able to stimulate photo-fenton chemistry through the reduction of iron bound to the phenolic ring via the pi electrons.

The use of an epifluorescent microscope with multiple wavelengths of light to stimulate the reactions enabled video analysis to be performed to determine the process of photo-fenton chemistry in manuka honey isolated Bee Product Extract.

Having established the potential of the Bee Product Extract to form Hydroxyl radicals using light and the photo-reduction of iron bound to pi electrons in the aromatic rings of phenolics present in the royal jelly Bee Product Extract isolated from manuka honey, a range of methods were used to attempt to characterize the product and its chemical reactivity.

Thus without wanting to be bound by a theory, the Bee Product Extract is able to perform photo-Fenton chemistry and generate Hydroxyl radicals and superoxides, which are responsible for the health benefits to the individual when used in a topical spray on the skin, nasally for respiratory conditions, orally for mouth conditions. The invention has identified that the Bee Product Extract when used in a topical spray (mist) formulation provides health promoting benefits that support vitality and well-being and has demonstrated all the functional benefits of manuka honey and Bee Product Extract combined.

The health-promoting benefits of proteins isolated from Manuka honey through the generation of Hydroxyl radicals through a photo-reduction of mono-atomic iron (Fe3+ to Fe2+) and copper (Cu2+ to Cu+) and other minerals whereby the reaction to endogenously produced hydrogen peroxide within the human body results in Hydroxyl radical generation. The said proteins isolated from Manuka honey, Bee Product Extract promotes apoptosis (pre-programmed cell death) and cellular regeneration.

Human beings emit electromagnetism and therefore can alter the chemistry that occurs within this system and as the speed of the reaction is faster than the eye can see and at a scale the eye cannot see and at a wavelength an eye cannot see these events would not be able to be observed with the naked eye within the human body. This is somewhat in the realm considered as quantum biology. However, upon application of the product increased energy within the body is obtained, increased mental clarity and increased vitality obtained, which has been documented. A model has now been developed to explain how it works and clinical evidence clearly backs up the model.

The functional benefits to healing and well-being attributed to the additional energy available to the human being provides an understanding of vital energy within the body originating from high energy short lived electrons. The generation of superoxide and Hydroxyl radicals and the support of cellular death through apoptosis, provides evidence for the regenerative properties of Manuka honey and in the extract produced by the manufacturing methods outlined in this specification.

The current understanding in the health and well-being market touts the detrimental effects of radicals on well-being, however, this is incorrect as they are elevated in the body to overcome the over use of high purified antioxidants. The radical based energy system in the human body is a primordial energy system in the body and developed early in cellular biochemical evolution. This system has regeneration functionality (apoptosis and is a cellular recycling system to stop damage accumulation in cells), is paramagnetic and is based on quantum entanglement, a form of electron communication at a distance and provides a deeper quantum communication system within our bodies. Loss of this quantum communication system in our bodies is proposed to lead to cancer formation, where a cell starts to grow independently and fail to communicate and no longer undergo apoptosis. Without the normal regulatory controls because of the loss of this communication it is understood that a wide range of diseases will be created in the human body.

There are a number of apalbumin glycoproteins that are found in honey and in royal jelly. The major apalbumin found in honey is Apalbumin 1 (Apal) also known as Major Royal Jelly Protein 1 (MRJP1). While the specification focuses on a Bee Product Extract from manuka honey, it is to be appreciated that the other apalbumins found in honey may also exhibit similar modification potential and similar biological activities due to the reactions that occur between protein-phenolics-mineral complexes and coordinated minerals in the generation of Hydroxyl radicals, via aromatic ring electron capture (K+) that react with the aromatic ring to generate DHA and MGO through iron coordinated (an other minerals such as copper and cobalt) photo-reduction to allow reaction with hydrogen peroxide and generation of the Hydroxyl radical and other high energy short lived radical species.

Photo-reduction of iron is defined by the capture of a single electron and transfer of this to the coordinated mineral bound to the aromatic ring structures in the phenolic compounds. Reduction of iron from Fe3+ to Fe2+ is also known as K+ or electron capture. The reaction of reduced iron (Fe2+) with hydrogen peroxide is outlined to generate Hydroxyl radicals and is part of the photo-Fenton chemistry occurring within Bee Product Extract to generate therapeutic benefits to individuals by supporting cellular death pathway called apoptosis, which supports cellular regeneration.

Subconscious mind physics is defined as the atomic physics performed by single atoms coordinated in neurotransmitters and providing the photons of light that create Balmer lines (hydrogen electron transitions) that are visible wavelengths of light that the human retina are able to absorb and cause a change in conformation of retinyl (cis to trans) which enables an electrical signal to be produced in neurological tissue enabling vision.

The modification of proteins by MGO occurs at Lys, Arg and Cys making the protein less allergenic due to the lysine residues being blocked and the lysine epitopes being responsible for allergenic potential of MRJP proteins.

Known composition of Manuka honey proteins include an array of proteins that have been identified by MSMS analysis that include MRJPs, apisimin and defensin 1 and a range of other bee proteins and plant proteins that have been identified in honey. The majority of the proteins are MRJP1 / apisimin complexes. Protein are known to contain phenolics and minerals that connect to the proteins through a range of chemical interactions, including but not limited to electrostatic, hydrophobic, pi stacking, metal affinity interaction, hydrogen bonding.

Sequences of major royal jelly proteins and apisimin have been reported in the literature. In the sequence of Major Royal Jelly Protein 2, 31 Lysine residues are present that can potentially react with MGO making the proteins less reactive towards IgE reactivity.

The relative volume of a protein occupied by its aliphatic side chains is termed as Aliphatic index (AI). In apisimin, the aliphatic index of cytotoxins in the range of 66.5 to 84.33 indicated that these proteins are thermally stable as well as they contain high amount of hydrophobic amino acids.

### Allergenic properties of royal jelly proteins

Studies have demonstrated that the major IgE-binding proteins of royal jelly were proteins between 32 to 67 kD corresponding to MRJP 1 and MRJP 2. This finding shows that MRJPs are important in allergy to royal jelly among individuals. The MGO modification of lysine regions within MRJP 1 and 2 are therefore potentially responsible for the reduction of the allergenic properties of these proteins within Bee Product Extract generated using the methods described in this invention.

Sequences of lysines in epitopes that are potentially causing allergenic properties as outlined in the allergome online database. http://www.allergome.org/script/dettaglio.php?id molecule=7627 MRJP1 and MRJP2 have been identified as allergy causing proteins in royal jelly.

the long term preparation of the Bee Product Extract from manuka honey provides ample time for MGO modifications of C, K, R residues in MRJPs and in apisimin bound to the resin during the solid phase of the process. These modifications reduce the allergenicity of the proteins making them suitable for use by people who typically react to the allergenic properties of royal jelly proteins.

### Hydrophobicity of honey and apisimin

| **Table 1. Hydrophobicity index of MRJP1 and apisimin** | |
|---|---|
| MRJP1 | Apisimin |
| Aliphatic index: 81.86 | Aliphatic index: 149.63 |
| | |
| Grand average of hydropathicity (GRAVY): -0.429 | Grand average of hydropathicity (GRAVY): 0.887 |

As can be seen by the hydrophobicity indexes for MRJP1 and apisimin, apisimin is considerably more hydrophobic than MRJP1. Apisimin is playing a role in the hydrophobic properties of honey and thus may play a role in the thixotropic features of manuka honey, and the recovery of the protein the process of the invention.

Methylglyoxal or MGO is a highly chemically reactive compound with the formula C3H4O2. MGO is formed by multiple metabolic pathways in living organisms. Certain preparations of manuka honey, which are referred to as "active' manuka honey, contain much higher concentrations of MGO than other varieties of honey. Active manuka honey has been determined to contain MGO concentrations up to 1000-fold greater than the MGO concentration in other varieties of honey (E. Mavric et al., 2008). MGO can participate in a variety of chemical reactions in living organisms, including the formation process of "Advanced Glycation Endproducts" (AGEs). Glycation is the reaction of a Sugar with a protein or a lipid without the involvement of an enzyme as a catalyst for the reaction. MGO can glycate proteins by reacting with the free amino groups of the amino acids arginine, lysine and/or cysteine and the terminal amino group, and thereby can chemically modify proteins that contain arginine and/or lysine. As can be seen from SEQ ID NO: 1 Apal contains a total of approximately 39 arginine and lysine residues that may be chemically modified by MGO.

### Therapeutic Formulations

Manuka honey Bee Product Extracts of the invention, and variants thereof having a different mineral composition due to the selection of acid and/or base in the production process, may be included in therapeuticallyeffective amounts in pharmaceutical compositions. The pharmaceutical compositions of the present disclosure may be specially formulated for administration in liquid form, including those adapted for the following: (1) oral administration, for example, drenches (aqueous or non aqueous solutions or Suspensions), e.g., those targeted for buccal, Sublingual, and systemic absorption, boluses, for application to the tongue, gums, teeth, throat and nose, and respiratory system through inhalation of mist or spray, (2) parenteral administration, for example, by Subcutaneous, intramuscular, intravenous or epidural injection as, for example, a sterile Solution or Suspension, or Sustained release formulation; (3) topical application, for example, as a cream, ointment, or a controlled release patch or spray applied to the skin; (4) intra vaginally or intrarectally, for example, as a pessary, cream or foam or spray; (5) sublingually; (6) ocularly; (7) transdermally; (8) pulmonarily, or (9) nasally. When the extracting of the present invention are administered as pharmaceuticals, to humans and animals, they can be given per se or as a pharmaceutical composition containing, for example, about 0.001 to 99%, or about 1 to 50%, or about 10 to 40%, or about 10 to 30, or about 10 to 20%, or about 10 to 15% of active ingredient in combination with a pharmaceutically accept able carrier.

The formulation can have added to it antioxidants and additional minerals can also be present in the pharmaceutical compositions described herein. Prevention of the action of microorganisms upon the compounds of the present invention may be ensured by the endogenous production of Hydroxyl radicals through the photo-fenton chemistry in the presence of a small amount of glucose and active glucose oxidase or through the addition of a small amount of hydrogen peroxide exogenously as well as exposure of the product in glass bottles to light of specific wavelengths corresponding to the absorbance of the phenolic compounds and production of photo-Fenton chemistry.

In the present invention the active ingredient Bee Product Extract from manuka honey is around 1 mg/mL when freeze dried and contains at least 1 billion proteins per mL and other preparations have been made that contain up to 10 billion particles per mL. The process of isolation being suitable for industrial production for mass production with widespread therapeutic benefits identified through human clinical investigation of the product produced in this invention.

### Analysis methods

The difficulties detecting MRJP1 complex using spectroscopy at particular wavelengths means that nontraditional approaches have to be used to detect the protein complex such as zeta sizer, qNano (Izon) and Nanosight. SDS PAGE analysis and Coomassie staining also failed to detect apisimin due to protein modifications. The wavelength of 280 nm is used to detect aromatic rings of Phe, Tyr and Trp. However, the 280 nm wavelength is larger than the entire MRJP complex at 11.5 nm x 10.0 nm. So the entire complex is smaller than the wavelength of light used to detect it making it difficult to detect and therefore quantify the amount of colloidal royal jelly protein present in Manuka honey. Optical microscopes have a diffraction limit of 200 nm and as the mrjp complex is smaller than this then the protein particles in honey when dissolved in water are practically invisible making their purification next to impossible. Their hydrophobic nature also makes then stick to surfaces and the chemical reactivity makes them change composition making analysis highly complicated. All of these characteristics have made the scientific investigation a challenging task and due to the variability and composition a complex analytical challenge. That is the nature of natural products recovered by bees in many different environment making each batch different from the last and therefore the variation adds to the complexity.

Methods of analysis of Bee Product Extract are outlined in this invention and can be performed using a number of appropriate methods using specialized instruments that can detect particles in the nanometer size and this has only been possible with the invention of new instrumentation that can detect nanoscale protein complexes. Another unique property of the Bee Product Extract prepared in this invention is due to the presence of phenolics and minerals and how these interact with light (photo-reduction) and promote photo-Fenton chemistry. The photochemistry is difficult to measure because the light used to measure becomes part of the process through which the reactions are generated causing changes in the products present in the Bee Product Extract produced by this process. This light sensitivity and reactivity is taken advantage of in the generation of the wide range of therapeutically beneficial ingredients that are present within Bee Product Extract produced in this invention.

The iron and copper bound minerals are capable of being photo-reduced and react with hydrogen peroxide to generate Hydroxyl radicals. This causes a considerable amount of energy to be released and the transformation of the material into something else. The use of light can cause transformation of Bee Product Extract. This complex system therefore needs to be handled appropriately to retain its functionality and bioactive benefits. This has now been achieved in this invention.

### Therapeutic uses

### Neurological benefits of Bee Product Extract

The effects of honey on brain health has been reported by others and attributed to the plant based antioxidants present in the honey. Rahman et al. reviewed the range of potential benefits honey has on the mind *(Evidence-Based Complementary and Alternative Medicine 2014,* Article ID 958721).

The effect of royal jelly ingestion for six months on healthy volunteers has been studied by Morita et al. Six-month ingestion of RJ in humans improved erythropoiesis, glucose tolerance and mental health. (Nutr J 2012 Sep 21;11:77).

In the present disclosure, memory is defined as the quantum tunneling of photons of light from hydrogen from the NH3+ amine functional groups into the monoatomic mineral coordination complex allowing the production of atomic isotopes. The origin of the hydrogen coming from the water produced in the body, that originates from food. The formation of memories from photons into biological structures within the biology of the individual using the physics of monoatomics.

The Bee Product Extract of the invention was shown to contain a number of phenolic compounds and these were covalently attached to apisimin when analysed by MALDI TOF MS. No matrix ions were used in the analysis and the natural phenolics within the product were used to absorb the MALDI laser which emits at 337 nm.

The role of phenolics is clearly outlined in the above scientific literature. However, the mechanism for memory has not been identified as the functional relationship between all of these compounds have not been recognized in relation to the functional role of neurotransmitter outside of the neurological firing system. The interaction across axons and the depolarisation events is a focal point of neurotransmitter function that has been talked about in the scientific literature. However, there is a more fundamental role of the neurotransmitter in the functioning of the mind at the level of the subconscious and that is the role of pi electron coordination chemistry with minerals (single atoms) also known as monoatomic mineral complexes with neurotransmitters.

The functional role of the phenolics is therefore due to complexes with minerals and how the monoatomic systems operate using light. The complexes found in Manuka honey performing photo-Fenton chemistry are synergistically doing exactly the same physics as our own neurotransmitters whereby the functional role is to add hydrogen into the monoatomic from the amine functional group (NH2 going to NH3+) and this creates an isotope of the monoatomic mineral coordinated to the neurotransmitter or to the phenolic in Manuka honey. These isotopes then decay releasing more energy and restore geometric balance in the atoms structure by producing an even number of protons and electrons and neutrons and positrons. This new understanding provides a rationale basis for the role of the product produced has in supporting the subconscious mind physics that occurs within the mind outside of the neuron firing systems.

The product, contains monoatomic minerals coordinated to the pi electrons and these are bound to the MRJP proteins that occur in the nanometer scale. This allows topical delivery and the widespread benefits of the monoatomic mineral system to support the subconscious minds function allowing increased energy using both radical (OH*) and atomic energies within the subconscious mind. The beta plus and beta minus decay processes are release photons of light as the quarks flip to generate an even number of up and down quarks. This model provides an understanding where light within dreams originates from and also supports the concept that atomic balance is restored during sleep without the conscious observer being present. So the subconscious mind is determined to be light based and the transitions of electrons and positron pairs in Balmer lines corresponds to visible wavelengths of light that the human's inverted retina (rods and cones on the inside of the eye) are able to pick up for the conscious mind to therefore observe and interpret.

The discovery of the nootropic bioactive in Manuka honey as originating from a photo-Fenton chemistry and the production of Hydroxyl radicals has led to a new understanding of the mono-atomic mineral system functioning in the brain outside of neurological processes. The coordination of single atoms to pi electrons in neurotransmitter aromatic rings has been shown to have a role in the subconscious mind physics and the generation of memory associated with quantum tunneling of hydrogen from amine NH3+ functional groups of dopamine and serotonin neurotransmitters. The Bee Product Extract from Manuka honey can be used topically or by other methods of administration to provide therapeutic benefits in a range of mental healthcare applications including Alzheimer's disease, bipolar disorder, brain fog, depression, anxiety and produces increased mental clarity.

The model itself is based on singularity physics corresponding to minerals coordinated to pi electrons in the phenolic aromatic rings where the quark number is changed to UP -1 and down +1 and multiplication is used to calculate the overall change in the subconscious mind. Whereas the conscious mind aspect which science has developed is based on adding fractions. The zero field of light (electromagnetism) is able to be observed as the neutron has a charge of -1 which is made zero by the positron +1 and the pairing between electron -1 and proton +1 also creates a zero. The zero being present when the atomic structure is in a geometry symmetrical state which is observed in light known to have not mass and no charge. This is what occurs before interaction and before measurement and the essential state the universe returns to when no other forces are at influence and a point of the singularity in atomic structures, as all that remains is zero, no contrast and therefore no beginning and no end. Without contrast or motion then there is no charge, no mass and no time. This occurs at the Planck Epoch.

The therapeutic properties of bioactive compounds present in honeybee products were reviewed by Cornara et al (Front Pharmacol. 2017; 8: 412. Benefits outlined included longevity (anti-aging), anti-inflammatory, neuromodular (brain health), and immunomodulatory benefits, and prevention of metabolic syndrome. The authors discussed therapeutic properties of an extract from Italian multifloral honey; 10-HDA and 10-Hydroxyldecanoic acid; royal jelly fatty acids; and Apamin.

### The production of superoxide using UVA light

The general protocol for analysis of superoxide production is as follows. The Bee Product Extract was tested for superoxide generation by adding 100 µL of the sample with 10 µL of 6.4 mg/mL NTB prepared in water. The samples absorbance was measured using SpectraMax M3 plate reader at 590 nm. The 96 well UV transparent plate was illuminated in Gel documentation system using a UV transilluminator set at 365 nm for 1 minute intervals and then measured again at 590 nm. The difference in absorbance before and after exposure to UVA light was determined.

The protein flocculated material was diluted in 10 fold serial dilution in DMSO and then 100 microL of sample was analysed for superoxide production by adding 10 microL of 6.4 mg/mL of NTB in water. The samples were then measured at 590 nm before UVA exposure and then again after 1.5 and 3.0 minutes UVA exposure. The background absorbance was removed from the measurements which were performed in triplicate from two separate experiments.

### Examples

### General protocol for batch methods using SP cation exchange resin

Manuka honey and a cation exchange medium are combined with potable water by vigorous stirring, in a ratio of 100 g : 100 g : to 1 L. The preferred cation exchange medium is SP Cellulose HG2, (Invitrogen), although as discussed herein, other media can be used. The pH is adjusted with a suitable acid, preferably a food grade acid such as H₂SO₄, to a pH of between about 5.0 and about 2.0 preferably below 4.5, more preferably in the range of 2.5 - 3.0, being a pH below the isoelectric point of the proteins in the solution. This step is preferably conducted quickly as the honey will start to react once it is dissolving. After 5 minutes the mixture is filtered. Optionally a cloth filter (such as a food grade Hop Bag) can be used for the filtering step. The resin was then mixed with an equal volumes of water and a base such as KOH added, to bring the mixture to a pH above the isoelectric point of the proteins, preferably to above 5.0, for example above 6.5, preferably between 8.0 and 10.5. The resin was filtered off and squeezed dry using a press, to recover the product of interest. Preferably, pH adjustments are made using food grade acids and bases. Subsequently, the pH can be adjusted to 6.5 to 7.5, making it compatible with skin and suitable as a spray formulation and topical application.

In selecting an appropriate resin any strong cation exchange resin can be used. Preferred are SP resins having sulphopropyl or p-toluenesulfonyl residues. The SP resin (Invitrogen SP HG2) was used in a batch format without the use of a column to avoid the aggregation issue associated with royal jelly insoluble aggregate formation. The sulphur propyl or p-toluenesulfonyl ligand of the SP resin is negatively charged and the royal jelly proteins in manuka honey are positively charged below their isoelectric point. The presence of MGO and DHA in the honey results in modification of the lysine and arginine residues, therefore the water diluted manuka honey with MGO present can result in modification of lysine and arginine residues therefore preventing interactions via ion exchange between the resin and the proteins.

In some embodiments a small amount of sugar is retained in the product and can react with the active glucose oxidase that is present in the honey to generate hydrogen peroxide. This reacts with the photo-reduced iron and copper in the product, thereby providing sterilization and preventing the need for additional preservatives.

While it is an advantage to use an MGO containing honey such as manuka honey, this is not essential. Advantages include that the MGO react with proteins in the honey providing allergenic benefits. It also reacts with glucose oxidase and inhibits production of H₂O₂. Honeys without MGO contain more H₂O₂ which affects the photo-Fenton chemistry occurring in the system and alters the final product.

### Example 1: Bee Product Extract preparation from manuka honey

Manuka honey (Egmont honey), 1 kg was dissolved into 10 L of potable water and placed into a 20 L plastic vessel. Then, 1 kg of resin SP cellulose HG2 (Invitrogen, Nelson resin plant) was mixed with the manuka honey solution for 5 minutes. The pH was measured was approximately 5.0. The pH was adjusted to 2.5-3.0 using concentrated H₂SO₄. When the pH had stabilized at pH 2.8 the resin was filtered off using a filter press. The resin was essentially squeezed dry under these conditions with a brown discolouration associated with the bound proteins. The damp resin was then placed into a clean 20L vessel and 5L of water added. The pH was then adjusted to 10.5 using 30% KOH. The solution was mixed and the solution filtered using the filter press as before to remove all traces of free water. The solution recovered was brown and contained the extract of interest. The pH was then adjusted using concentrated H2SO4 to a pH suitable for topical application to the skin between 6.5 to 7.5.

This method was used to produce the Bee Product Extract. The contact time of the manuka honey with the resin can be months to allow germination of the pollen present in the honey, or maturation processes whereby the released components of the pollen react with the bound royal jelly proteins to enable modification to the proteins and reduce their allergenic properties. The dried resins was returned back into the original manuka honey solution for another round of protein recovery.

The cycle and recovery of protein from manuka honey using this method was found to be repeatable over at least five cycles. A month or more could be used between batches. As the presence of glucose oxidase, sugar and phenolics and minerals in the Manuka honey was such that complexation chemistry and chemical reactivity was occurring during this period, which resulted in the formation of new active ingredients that were bound to the royal jelly proteins which under conditions below the pl of the proteins bound to the resin. The chemical reactions created further active ingredients which was then recovered using the method outlined above included centrifugation, dissolved air floatation, suction filter, gravity filter and rotary vacuum filtration methods. The approach avoids column methods due to the issues discussed below regarding royal jelly proteins. As mentioned above, the current state of the art in protein purification using chromatography uses columns. A batch processing method was used in the above example to take into account the complexity of nanoprotein particles in manuka honey having thixotropic properties, and the tendency of MRJP1 to form long polymers under acidic conditions whereby the proteins are not able to bind to the resin and end up forming complexes that are too large to purify. This results in resin fouling when the resin is in a column. It also results in lower yields and it results in reduced resin life expectancy (number of resin cycles) before the resin has to be thrown away. This increases costs and makes the process uneconomical.

The art also teaches that resins should not be allowed to dry within a column under such conditions channels occurs which reduces the recovery of protein eluted from the resin in a column.

### Analysis

### Reaction with Nitrotetrazolium blue (NTB)

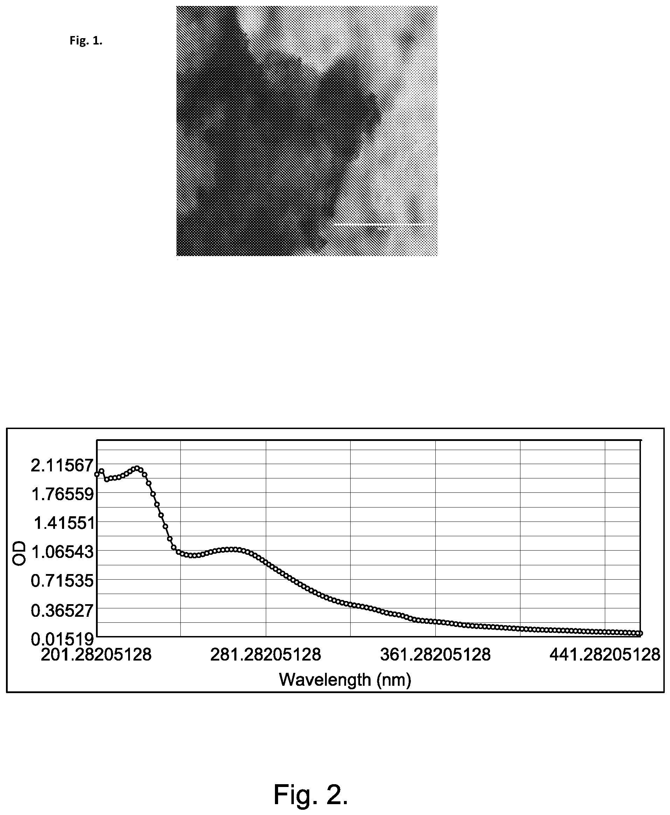

Figure 1 depicts the NTB reaction with manuka honey under a microscope exposed to UVA light to enhance the generation of super oxide radicals through photo-Fenton chemistry. The precipitate generated during light exposure under the microscope provides the evidence for light mediated iron photo-reduction and generation of NTB reactive species.

### UV/Visible absorbance spectra

The absorbance of the Bee Product Extract from manuka honey was measured from 200 to 1000 nm using a SpectraMax M3 spectrophotometer (Figure 2). The main absorbance peak was around 260 nm corresponding to phenolic compound absorbance. No absorbance peaks were observed at wavelengths greater than 440 nm. Due to the small size of the MRJP protein complexes (100 nm) UV / Vis methods fail to detect the protein as absorbance at 280 nm used in protein chromatography methods fails to detect much of the colloidal nanoparticle proteins present in Manuka honey. Therefore, the standard methods used for detection are not useful for identifying the nanoparticle complexes present in Manuka honey. These properties means the protein content in manuka honey is underestimated and alternative approaches are outlined in this specification as to more appropriate methods to use to analyze Bee Product Extract produced in this invention. , showing the aromatic absorbance based on phenolics present in the Bee Product Extract.

### SDS PAGE protocol and analysis

Samples 20 µL were mixed with 5 µL of 4 times LDS sample buffer vortexed and then 20 µL loaded onto the gel. No reducing agent was added to the samples and they were not boiled.

NuPAGE Bis Tris 4-12% gradient gels (ThermoFisher Scientific NP0321BOX) were employed in this study. The gels were stored at 4 °C before use and prepared for use by removing from the plastic bag and removing the tape covering the foot of the gel and the comb making sure the wells were free of extraneous gel material before placing into the gel tank. Fresh MES buffer was prepared 50 mL into 1 L of MilliQ water and internal and external chambers of the gel tank filled with buffer to the desired level which covered the wells and foot of the gel. Then the standard 10 µL and samples 20 µL were loaded onto the gel. The gel was run at 140 mA/gel using 200V at room temperature. The gel was typically run for 28 minutes or until the dye front reached the foot of the gel whilst still retaining the lowest standard (3 kDa) in the gel. The gel was then removed from the buffer tank and the plastic plates removed using a gel knife. The gel was either stained with Coomassie R250, silver stained or immunoblotted using an apisimin antibody.

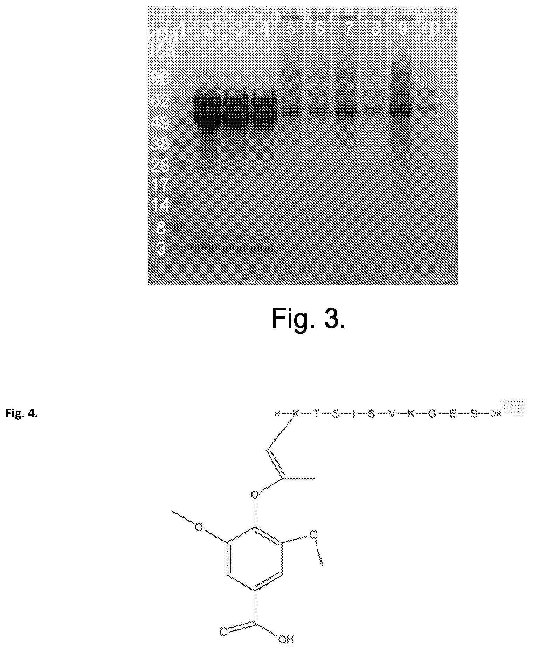

Figure 3 shows SDS PAGE and coomassie staining of various bee product compositions. The Protein MW markers are in Lane 1, while MRJPs present in royal jelly are in Lanes 2-4. Lane 5 contains the Bee Product Extract of Example 1 (prepared from Egmont manuka honey), while lanes 6 - 10 contain Bee Product Extracts prepared using the same protocol as Example 1, from different manuka honeys. Figure 3 demonstrates the inability to stain apisimin using coomassie G250. This is due to the lysine residues in apisimin being blocked through MGO modification in the production of Bee Product Extract, thus making it functionally different from royal jelly apisimin which is not modified by MGO or phenolic adducts.

The art of protein chromatography also teaches the use of salt based elution methods and downstream processing using ultrafiltration methods including nanofiltration. These additional steps cost time, money and affect yield making the product less economically viable and therefore unable to be scaled up to industrial scales for widespread marketing and distribution. These issues make recovery of the nanoproteins from Manuka honey ($60 / kg) unfavorable using column chromatography methods and therefore the art and current standard of knowledge regarding proteins in honey at 0.1 to 1% content is seen as not being of economic importance and there are no products currently in the market based on Manuka honey recovered MRJPs.

The resin SP cellulose is chosen due to the selectivity of the ligand to binding the target components within Manuka honey. It has an aromatic ring structure (SP ligand) that not only carries pi electrons which can coordinate metal ions via complexation chemistry (IMAC) immobilized metal affinity chromatography, but it can also perform pi stacking ring interactions with the phenolic aromatic rings within the phenolics bound to the proteins in Manuka honey. The negative charge on the resin is important for ion exchange type interactions. However, when salt is used to elute such proteins off the ion exchanger the proteins remains tightly bound because of the pi interactions, IMAC interactions and the hydrophobic pi stacking interactions all of which cause complexities when trying to purify the proteins from Manuka honey. To overcome such complex array of interactions the very minimal amount of processing is performed. Under these conditions the complexity of the protein nanoparticle complexes (colloidal proteins) is retained where the protein has also bound the phenolics and the minerals are coordinated to the pi electrons in the aromatic rings of the phenolics enabling photo-Fenton chemistry to be undertaken in the presence of light of particular wavelengths (absorbance by the phenolics). The transfer of the electron into the bound atom (coordination complex) provides a highly reactive Fe2+ which can react with hydrogen peroxide (generated by glucose oxidase present in the honey), which reacts to produce Hydroxyl radicals.. OH* being the active ingredient that performs the antibacterial, and antiviral properties in Manuka honey. The OH* being highly reactive turns biology back into CO2 and water and is part of the natural death and regeneration system present in apoptosis (pre-programmed cell death).

The role MGO (methyl glyoxal) has in antibacterial activity in Manuka honey is a correlation to antibacterial activity. MGO is broken down in the body by the glyoxylase pathway and therefore not therapeutically active in Manuka honey. Clinical work with MGO has clearly demonstrated its failure to account for the activity observed in Manuka honey. As a consequence it is just a substitute for the real active ingredient and that is OH* the Hydroxyl radical.

The presence of MGO in manuka honey has been shown to inhibit glucose oxidase. Therefore, hydrogen peroxide generation in Manuka honey has been inhibited through MGO reaction to glucose oxidase. This is why Manuka honey is known as a non peroxide antimicrobial honey (UMF and NPA corresponding to MGO levels determined by science and used by the Manuka honey industry).

The speed of OH* formation and its ability to turn biology into CO2 and water means that when honey is diluted the glucose oxidase produces hydrogen peroxide and the reduced iron and copper in the honey react to generate OH* by Fenton chemistry. This does not occur in Manuka honey, however, photo-Fenton chemistry does occurs.

MGO inhibits glucose oxidase and the inhibition means the MRJP-phenolic-mineral complexes are photo-reduced (iron in Fe2+) which is able to react with hydrogen peroxide. Hydroxyl radicals are not produced because hydrogen peroxide is not present. This allows the recovery of MRJPs intact using the method developed and explained in this specification enabling the production of a OH* generating system by have photo-reduced Fe2+ in a coordination complex with phenolics bound to MRJP proteins that are recovered from Manuka honey using the novel methods outlined herein.

The location of the phenolic could be located in the pocket formed by apisimin and occupied by 24 methylene cholesterol In the absence of 24 methylene cholesterol the pocket within the complex could accommodate a range of phenolic compounds with coordinated minerals. The MALDI TOF MS analysis of the Bee Product Extract revealed the formation of adducts between the N-terminus of apisimin and phenolic residues suggesting that the phenolics were in close proximity to the N-terminus. The structures available on the PDB database 5yyl protein 1 and 5yyl protein 2 have a truncated form of apisimin starting at residue D37 in the structures. The first amino acids in the sequence are not shown KTSISVKGESNV. The position of this peptide is therefore currently unknown. In the absence of the 24 methylene cholesterol it is proposed that the N-terminal peptide may reside within the cavity of the protein, however, in the presence small molecules including phenolics and 24 methylene cholesterol the structural position of the N-terminus remains to be elucidated. What is interesting as in the case for Manuka honey is that the exposure of the colloidal particle to MALDI TOF MS laser resulted in chemical reactions within the colloidal structure as the phenolic absorbed the laser light (without matrix addition). The reactions were analyzed by MS and MS/MS to reveal a chemistry that occurs to form adducts between MGO, phenolics and the N-terminus of apisimin leading to the production of novel compounds with protease inhibitory activity. The photo-reduction chemistry lead to rapid production of Hydroxyl radicals that connect the compounds together and generate the unique molecular structures within the MALDI TOF Ms instrument and it is proposed that similar reactions occur within cells generating phenolic-MGO-apisimin peptides adducts in the phagosome due to the reaction with chemical energy of hydrogen peroxide. Figure 4 depicts the phenolic-MGO-apisimin complex. The products from the MS analysis reveal the close proximity of the N-terminus of apisimin within the cavity of the colloidal complex of MRJP1-apisimin-24 methylene cholesterol which has x-ray structural data (without the N-terminus). The phenolics present in Manuka honey would appear to be held within the apisimin-MRJP1 complex and mono-atomic iron associated with the phenolic ring pi electrons. This enclosed environment when exposed to the MALDI TOF MS laser generated N-terminal truncated adducts.

### Nanoparticle analysis (qNano)

The Bee Product Extract of Example 1 was characterized for size and charge and number using a range of methods. As the proteins are in the nm scale, their direct measurement using UV / Vis spectrometry is difficult due to the size and geometry and the ability of the Bee Product Extract to perform Photo-Fenton chemistry which uses energy within the wavelengths of light use to determine absorbance to catalyse the production of reduced iron and if any hydrogen peroxide is present in the aqueous solution the generation of Hydroxyl radicals can occur and other high energy short lived radicals like superoxides. Analysis was performed using qnano device to determine the number of particles per mL and their relative size based on nanopore technology developed by Izon. Analysis was performed independently by Izon using the standard methods of preparation, as follows.

### SAMPLE AND REAGENT PREPARATION FOR QNANO ANALYSIS

1. Measurement Electrolyte: 2x PBS + 0.03% Tween-20 is used to prepare the sample and calibration particles.
2. lzon calibration particles (CPC100. nominal diameter of 100nm) were used as calibration particles.
3. Nanopore NPBO (Analysis range: 40-255 nm) was used. The nanopore was coated with the Izon Coating Solution which helps with system stability and prevents non-specific binding of particles to the nonopore.
4. Sample preparation:
   a. 1ml of sample was centrifuged at 10,000g for 10 minutes. Collect supernatant.
   b. Load 0.5 ml of supernatant onto qEVoriginal/35. 1.5 ml of particle-rich volume (fraction 7, 8, 9 after void volume) was pooled.
   c. Sample was then concentrated using Amicon Ultra-15 100 kDa.
   d. Concentrated sample was then diluted 1n Measurement Electrolyte(LS) and used in measurement.

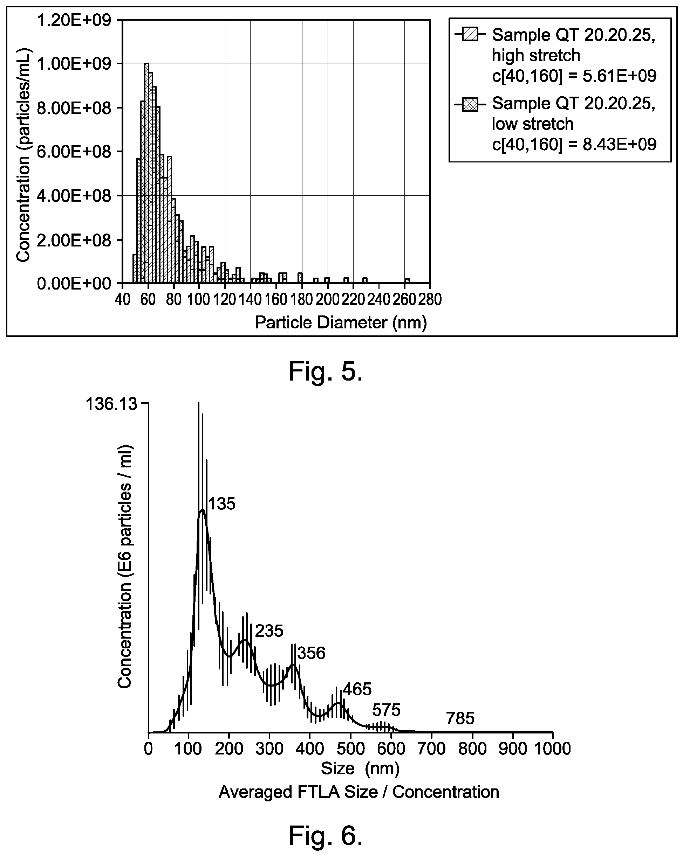

Manuka honey Bee Product Extract of Example 1 was analysed for proteins in a colloidal nanoparticle form using qNano instrument (Izon). The results are shown in Figure 5. External analysis was performed to validate the numbers of bioavailable royal jelly particles isolated. Over 1 billion particles per mL were produced using the novel manufacturing method developed. Analysis demonstrated typically between 5 to 10 billion royal jelly colloidal particles per millilitre were able to be purified by this method. The histogram of particle size distribution is shown in Figure 5, for the Bee Product Extract of Example 1.
The number of protein particles per mL in Bee Product Extract of Example 1 is shown in Table 2.

**Table 2: Analysis of protein particles number and size using qNano device**

| **Sample Label** | **Measurement Date** | **Mean Dia (nm)** | **Mode Dia (nm)** | **d90/d10** | **Raw Conc (/ml)** | **Particle Count** | **Particle Rate (/min)** |
|---|---|---|---|---|---|---|---|
| Sample QT 20 20.25, high stretch | 9/09/2020 3:57:23 PM | 84 | 76 | 1.7e+0 | 5.61e+009 | 233 | 23.3 |
| Sample QT 20.20.25, low stretch | 9/09/2020 4:52:37 PM | 70 | 65 | 1.7e+0 | 8.43e+009 | 387 | 38.7 |

### Nanosight analysis of isolated colloidal nanoparticles isolated from Manuka honey

Independent analysis of the Bee Product Extract as outlined in Example 1 was performed by the University of Auckland using a NanoSight N300 instrument. The NanoSight capture setting and analysis results for Bee Product Extract prepared from manuka honey are as follows.

| **Capture Settings** | | **Results** | |
|---|---|---|---|
| Camera Type: | SCMOS | Stats: Merged Data | |
| Camera Level: | 9 | Mean: | 230.1 nm |
| Slider Shutter: | 450 | Mode: | 133.1 nm |
| Slider Gain: | 250 | SD: | 118.8 nm |
| FPS | 25.0 | D10: | 109.2 nm |
| | | D50: | 193.0 nm |
| Number of Frames: | 749 | D90: | 392.1 nm |
| Temperature: | 25.4 - 25.4 °C | | |
| Viscosity: | (Water) 0.9 cP | Stats: Mean +/- Standard | Error |
| Dilution factor: | Dilution not recorded | Mean: | 229.9 +/- 8.3 nm |
| Syringe Pump Speed: | 50 | Mode: | 149.7 +/- 14.1 nm |
| | | SD: | 117.0 +/- 5.4 nm |
| **Analysis Settings** | | D10: | 106.6 +/- 7.9 nm |
| | | D50: | 194_5 +/- 12.5 nm |
| | | D90: | 393.2 +/- 29.7 nm |
| Detect Threshold: | 10 | Concentration: | 1.26e+009 +/- 1.20e+008 particles/ml |
| Blur Size: | Auto | | 64.0 +/- 6.1 particles/frame |
| Max Jump Distance: | Auto: 11.3 - 13.1 pix | | 102.8 +/- 6.2 centres/frame |

Figure 6 shows the Bee Product Extract from manuka honey of Example 1 has a wavelike particle size distribution. The monoatomic minerals coordinated to the pi electrons bound in the phenolic aromatic ring to respond to the laser light used in the Nanosight instrument, providing the wavelength transitions between positron and electron pairs within the mineral complexes as the photons cause the photo-electric effect in the light entanglement pairs of positron and electrons within the monoatomic atom system.

### Zeta sizer analysis

Zeta sizer analysis of the Bee Product Extract prepared in Example 1 determined the proteins were around 100-200 nm in size.

### MALDI TOF mass spectroscopy

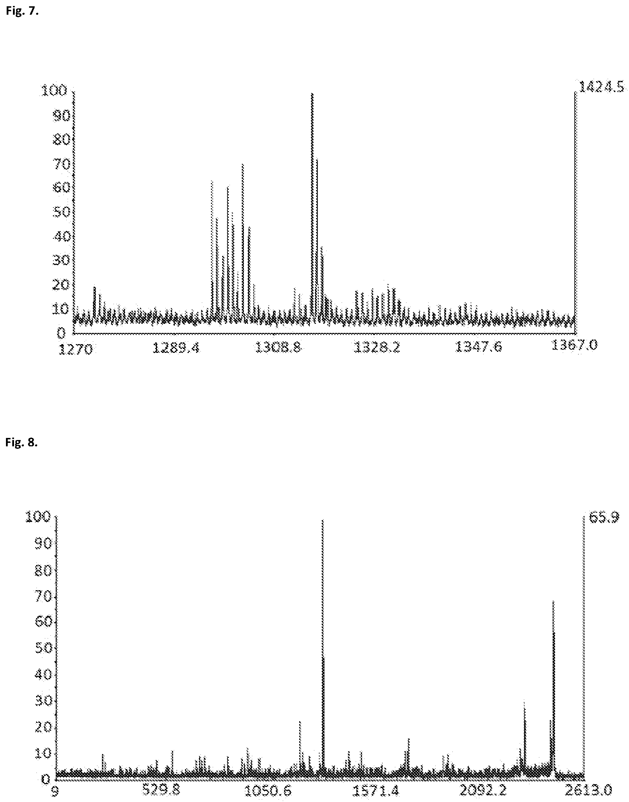

Figure 7 shows the MALDI TOF MS of the Bee Product Extract of Example 1. Figure 8 shows the MALDI TOF tandem mass spectrum (MSMS) of the apisimin fragment detected in the Bee Product Extract of Example 1, peak at 2472 Da and the identification of apisimin N-terminal peaks without the detection of lysine first amino acid in apisimin due to MGO phenolic structural modified during Bee Product Extract production.

### SCX HPLC

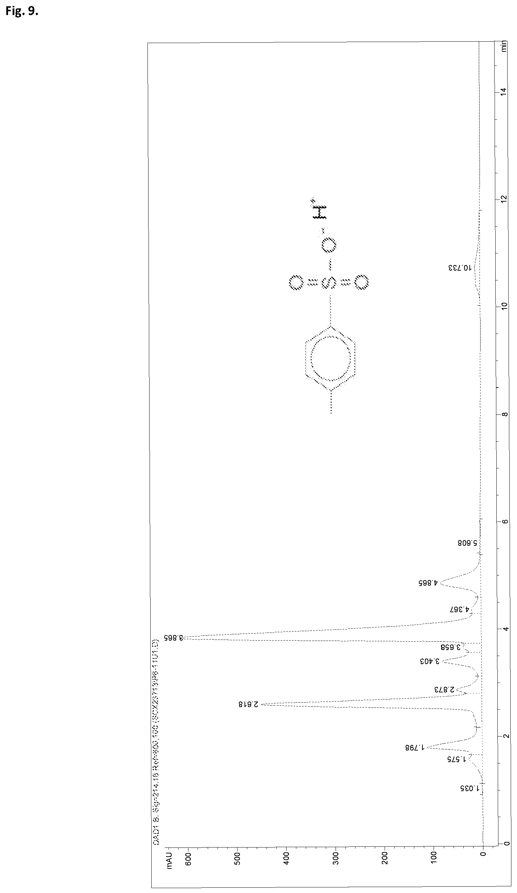

Figure 9 shows the SCX HPLC elution profile of Bee Product Extract of Example 1. Separation in water without modification of pH due to the coordination chemistry and not typical of ion exchange chromatography. When salt was added to elute the proteins the proteins remained bound to the resin material clearly indicating that ion exchange was not the mode of binding. The presence of pi electrons provides a HIC type or mixed mode interactions and the presence of minerals provides IMAC type of interaction. These novel interactions provided the power to resolve and separate the Bee Product Extract of the invention.

### Example 2: Dissolved Air Flotation (DAF) production of Bee Product Extract from manuka honey

In this example the Bee Product Extract from Manuka honey was produced using dissolved air flotation (DAF) where a fish bubbler was used to pump small air bubbles through a porous glass media to create a flotation system to recover proteins from dissolved manuka honey. A 5% final concentration sugar solution was prepared from manuka honey by dissolving the honey into water. The bubbler was added and the air pumped through to generate a white foam on the top of the fluid. The flocculant in the foam was then collected.

The Bee Product Extract of Example 2 was analyzed using a range of biochemical techniques including SDS PAGE, RP HPLC, FTIR, SEM, MALDI TOF MS, zeta sizer analysis and composition of phenolics and minerals using EDS methods after TGA analysis. The complex array of molecules present suggests that the Bee Product Extract appears to be colloidal in nature and are typically around 100 nm in size, and are significantly modified by phenolic adducts via chemical covalent linkages that are formed through MGO based chemical reactions to a number of amino acids in the various proteins present in manuka honey. The presence of minerals including iron in coordination complexes has been demonstrated to produce a range of radicals including superoxide and the hydroxyl radical which have functional role in the biological activity within the Bee Product Extract.

The properties of Manuka honey, being a non peroxide antibacterial honey due to MGO inhibition of glucose oxidase (Majtan J, Bohova J, Prochazka E, Klaudiny J. "Methylglyoxal may affect hydrogen peroxide accumulation in manuka honey through the inhibition of glucose oxidase". J Med Food. 2014;17(2):290-293. doi:10.1089/jmf.2012.0201 ). The antibacterial properties of honey has been attributed hydroxyl radical generation (Brudzynski K, Lannigan R. "Mechanism of Honey Bacteriostatic Action Against MRSA and VRE Involves Hydroxyl Radicals Generated from Honey's Hydrogen Peroxide". Front Microbiol. 2012;3:36. Published 2012 Feb 7.), and the presence of phenolics and hydrogen peroxide in honeydew honey's (Bucekova M, Buriova M, Pekarik L, Majtan V, Majtan J. "Phytochemicals-mediated production of hydrogen peroxide is crucial for high antibacterial activity of honeydew honeys". Sci Rep. 2018;8(1):9061. Published 2018 Jun 13.). The bacterialcidal effects of honey have been seen to be associated with DNA degradation (Brudzynski K, Abubaker K, Wang T. "Powerful bacterial killing by buckwheat honeys is concentration-dependent, involves complete DNA degradation and requires hydrogen peroxide". Front Microbiol. 2012 Jul 4;3:242.) The role of hydroxyl radicals in apoptosis and the degradation of DNA provides a causative link. Hydroxyl radicals have been targeted as damaging to DNA, proteins and lipids and are suggested to be directly involved in cellular damage and aging. Therefore, suggesting that Manuka honey's health benefits are associated with the generation of hydroxyl radicals is highly controversial and goes against the majority of scientific literature of the past 60 years, and confronts the therapeutic benefits of an entire healthcare industry selling antioxidants. The fact that apoptosis is a natural process of biological recycling and converting a cell into CO₂ and water by the way of radical chemistry driven by hydroxyl radicals suggests that the regenerative properties and cell renewal are associated with radical chemistry as an energy system of cellular recycling or transformation, and a natural part of cellular biochemistry and part of cellular evolutionary processes. This makes sense as the energy released from radicals provides ATP independent chemical transformation and enables cells to die without costing energy to the entire organism, which would be considered a highly evolved successful strategy in evolutionary terms of cellular regeneration processes. Manuka honey has all the components for photo-Fenton chemistry except one, and that is hydrogen peroxide production due to glucose oxidase inhibition. The high concentration of iron and phenolics suggests that if a small amount of hydrogen peroxide was present within the colloidal protein complex then the generation of hydroxyl radicals may be responsible for the antimicrobial properties of Manuka honey. The isolation and characterization of Bee Product Extract from Manuka honey was undertaken to determine if their composition was relevant for the generation of photo-Fenton chemistry and the production of antimicrobial hydroxyl radicals and promotion of the regenerative capacity and healing properties known to be associated with manuka honey.

### Example 3: Dissolved Air Flotation (DAF) production of Bee Product Extract from manuka honey, overnight reaction

Honey was diluted into distilled water at ambient temperature then mixed to homogeneity with a spatula to produce a 2-5% sugar solution from the honey. The honey was loaded into a 2 L separating funnel. Air was bubbled gently through the honey solution and the bubbles that rose formed foam at the surface of the solution. The foam was observed to be white in colour and a white flocculent material ("white floc") could be recovered from this foam. The bubbling was allowed to proceed for several hours (overnight) on a low flow rate of air of 10 mL/min. At the end of this period, the supernatant solution of the honey was yellow in colour and transparent (having been cloudy at the outset) whilst the foam and top surface of the separating funnel was covered in brown particulate material and foam. The tap at the bottom of the separating funnel was opened and the 'clarified solution fraction' (CS) was recovered. 500 mL of distilled water was added to the separating funnel to recover the brown flocculent material ("brown floc") from the honey solution. Without wishing to be bound by theory, the colour change is thought to be caused by oxidation processes. As discussed below, unless otherwise stated, a reference to the Bee Product Extract of Example 3 is a reference to the brown floc. The absorbance profile for Manuka honey flocculated solutions containing Bee Product Extract of Example 3 was shown to contain proteins and phenolics. The clarified solution had a maximal absorbance at 260 nm and also appeared to contain phenolic compounds. All three samples absorbed at 280 nm and had a peak absorbance around 230 nm. The absorbance tailed off for the DMSO control around 270 nm, whereas the samples had considerable absorbance around 350 nm.

The white floc, brown floc and clarified solution were subjected to fluorescence analysis. The sample of the flocculated protein was analysed for fluorescent properties using Ex 280 nm and Em 330-600 nm using A325 nm cutoff filter. The Bee Product Extract, brown floc had an emission peak at 350 nm whereas the emission peak for the clarified solution was at 360 nm. All three materials appeared to emit light at 450 nm.

### Freeze drying

The Bee Product Extract, brown floc was recovered freeze dried. From 102.8 g of manuka honey was recovered 1.1 g of freeze dried Bee Product Extract. This suggested that around 1.07% of material was able to be recovered by the DAF process after an overnight process.

### Thermogravimetric analysis; EDS SEM analysis

The freeze dried product was then subjected to thermogravimetic analysis. 6.29500 mg freeze dried protein material was placed into the pan for TGA analysis. 12.6 % of the 6.295 mg remained as ash after burning off at 1300 °C. The material was taken from room temperature to 1300 °C at 10 °C /min in a stream of air. The recovery weight of the Bee Product Extract was 1.63 g from 500 mL of 20% Manuka honey, i.e. around 1.6%. The recovered proteins were hygroscopic.

The ash that remained after the TGA treatment was collected and analysed by energy-dispersive X-ray spectroscopy (EDS) analysis for mineral content using scanning electron microscope (SEM). There was a high iron content detected by EDS analysis. Calcium and phosphate was also observed (Table 3).

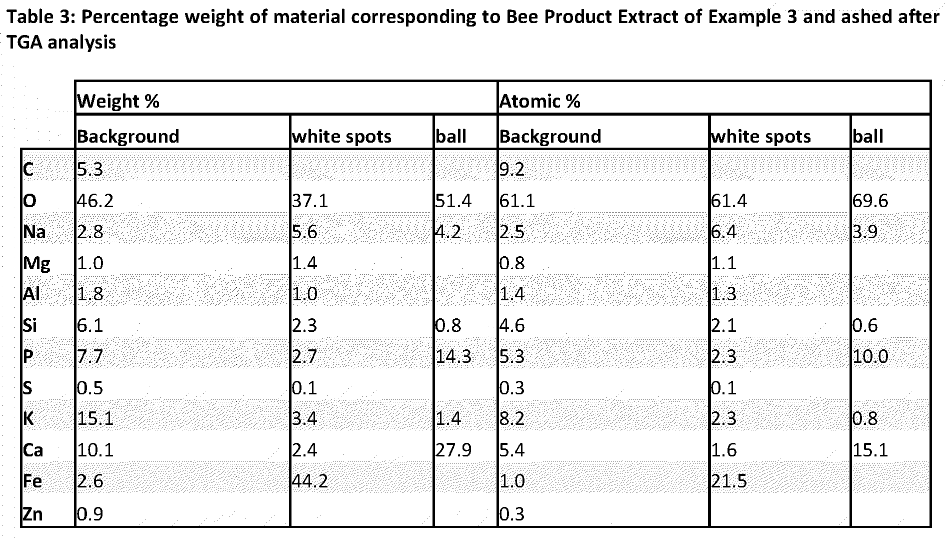

12.6% of the Bee Product Extract was ash. The balls were apparently a form of calcium phosphate and the background ash material had lower iron content. The reason for the high iron content in particular materials is unknown but it appears to be unevenly distributed throughout the sample after incineration.

### FTIR

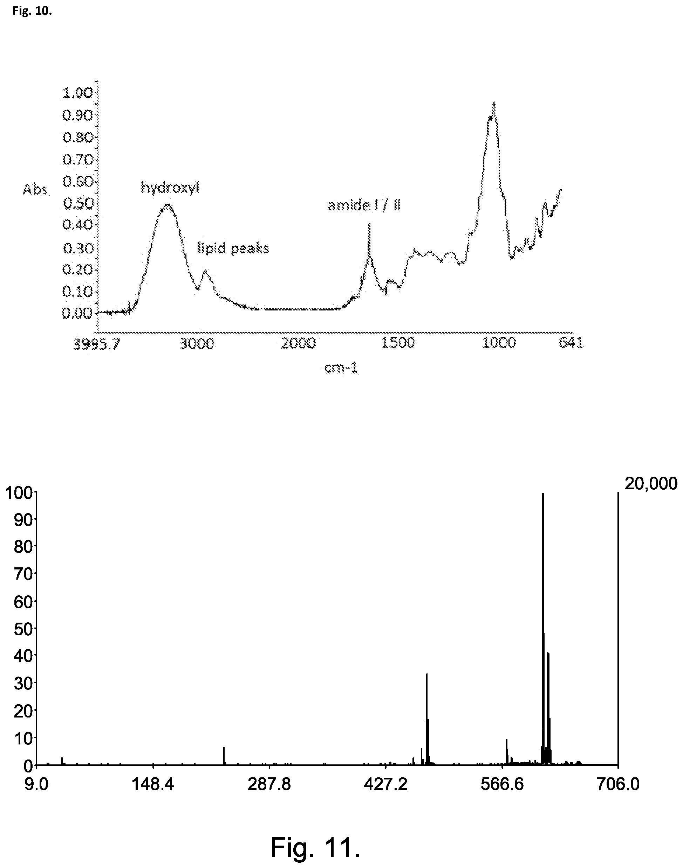

Figure 10 shows the FTIR spectrum of Bee Product Extract of Example 3 brown floc. The profile shows a complex array of molecular functionality that is produced using the Bee Product Extract DAF manufacturing procedure. The FTIR fingerprint shows the presence of the proteins.

### MALDI TOF MSMS analysis

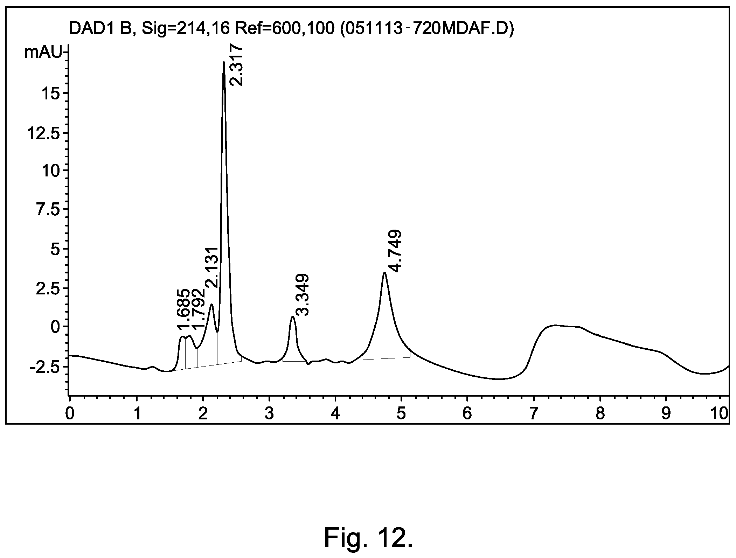

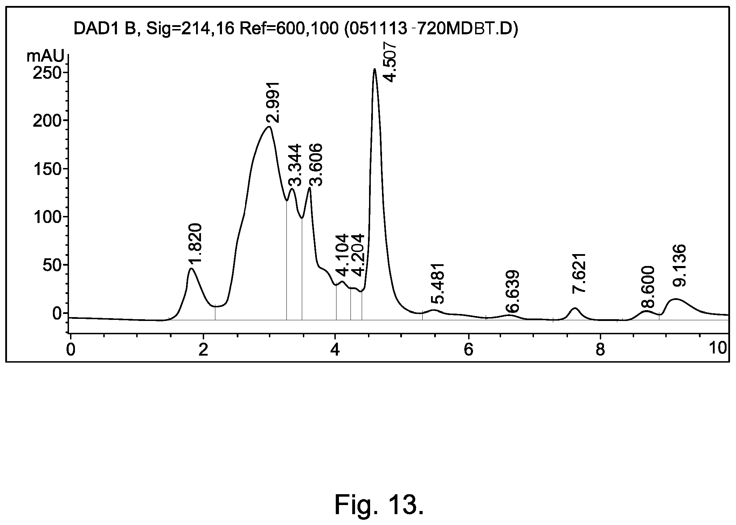

Figure 11 shows the MALDI TOF tandem mass spectrum (MSMS) Bee Product Extract of Example 3, peak at 667 Da and the identification of apisimin N-terminal peaks without the detection of lysine first amino acid in apisimin due to MGO phenolic structural modified during Bee Product Extract production. Within each region of the spectra there consists a molecular mass difference of 211-212 Da. There was also a 16 Da series of peaks. The difference of 212 may have been due to a phenolic compound. The cause for the difference of 16 Da mass units is unknown (16 mass units equates to one O atom or NH₂, but these peaks with 16 Da mass unit differences do not necessarily relate to one another as parent/daughter ion peaks). It is possible and reasonable that there are a range of phenolic compounds present, differing by one or more O atoms, which could readily give rise to such a MS pattern. Figure 12 depicts the SCX HPLC profiles of Bee Product Extract of Example 2 (white floc), while Figure 13 depicts the SCX HPLC profile of the clarified solution of Example 2. The profile changed due to the chemical reactions that occurred during the flocculation recovery process.

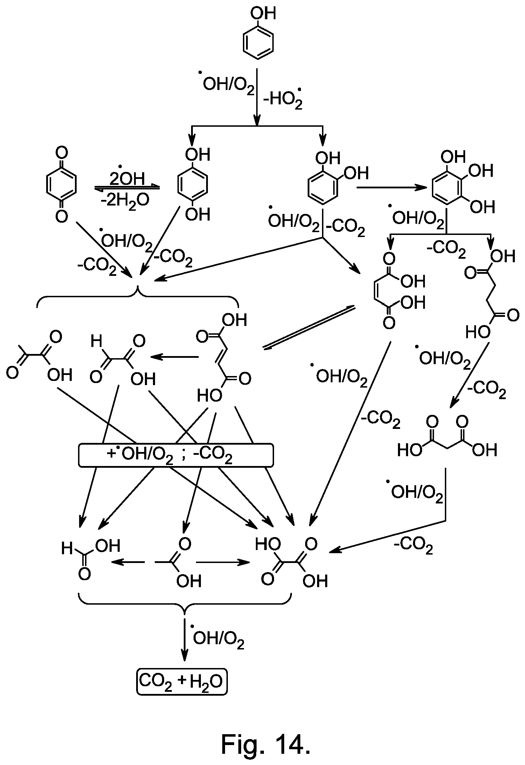

The UV/Visible absorbance spectrum did not appear to show any significant phenolics present. However, as the sample was from the overnight flocculation process and the sample had been exposed to light the presence of phenolics may be lost due to the generation of reactive species such as superoxide and the hydroxyl radical which can react with the phenolics to break them down into small compounds as outlined in Figure 14. The ability to make the vesicles soluble using DMSO was beneficial, but it was noted that the SCX HPLC profile changed and that A214 nm was no longer able to be used to quantify the amount of Bee Product Extract containing apisimin present because DMSO absorbed at this wavelength used to monitor this protein. The eluted protein HPLC trace shown above was determined at A260 nm. It is known that apisimin does not absorb at this wavelength and neither does DMSO. It is therefore believed that the profiles above in Figures 12 and 13 may be due to other compounds present in the sample such as phenolic groups associated with the protein or peptides generated during the process of production.

### Example 4: Superoxide production by Bee Product Extract from Manuka honey

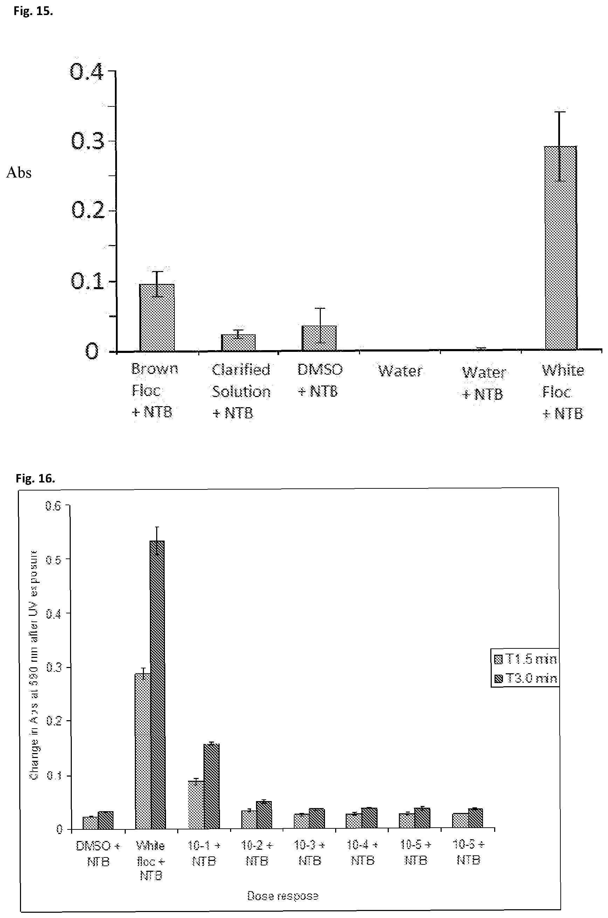

Bee Product Extract of Example 3 was also analyzed for the production of superoxide using nitro tetrazolium blue (NTB) after exposure to UVA light to determine the photo-Fenton chemistry potential of the Bee Product Extract.. The results are shown in Figure 15, and show the proteins recovered produce superoxide when exposed to UVA light, due to their composition of having minerals and phenolics bound to the proteins. It was also suspected that minerals were bound to the proteins but it was not expected that the minerals were also bound to pi electrons within the phenolic aromatic ring bound via coordination chemistry. This was later determined to be occurring and responsible for the generation of photo-reduced iron and the generation of hydroxyl radicals via photo-Fenton chemistry.

The use of UV light and the generation of reactive oxygen species was explored.

The absorbance of samples from Example 3 was measured after the addition of NTB and exposure to UV light. The samples were dissolved in DMSO, so an appropriate DMSO control and water controls were also used. Figure 15 depicts the results. The clarified solution did not appear to be able to produce superoxide whereas the recovered Bee Product Extract from Manuka honey (white floc and brown floc) produced superoxide when exposed to UV light. Figure 16 depicts the dose response for the Bee Product Extract Example 3, white floc, and production of superoxide of samples prepared in DMSO. A 100 fold dilution of the Bee Product Extract isolated from Manuka honey superoxide was produced in response of the material exposed to UV light. 1 mL was recovered and diluted to 5 mL of a 20% honey solution. The resulting 4% Manuka honey solution was diluted 100 times and still shown to still be able to produce superoxide when exposed to UV light. New Zealand with its thinner ozone layer has been exposed to higher levels of UVA and therefore the generation of superoxides within the Manuka honey may be higher than normal due to our light spectrum, clean air and our environmental conditions where manuka plants grow as a pioneering plant in soils with elevated iron content. All of these aspects making New Zealand's manuka honey unique in its ability to conduct photo-Fenton chemistry in the generation of hydroxyl radicals giving New Zealand's manuka honey its unique properties and its regenerative and anti-bacterial, anti-viral and anti-inflammatory properties.

### Example 5: Preparation of Bee Product Extract using continuous centrifugation

Shop purchased manuka honey (100 g) was dissolved in water (1L) to create a 5% sugar solution containing the royal jelly proteins, phenolics and minerals. The resin SP cellulose HG2 was added (100 g). The pH was adjusted to 2.8 with concentrated sulphuric acid. The resin was mixed for 10 minutes and then the resin in the dissolved manuka honey solution was added to a food juicer and spun for several seconds to remove the resin from the water phase. The resin was recovered and the bound protein recovered by adding water (1L) and adjusting the pH to 9.5-10.5 with 30% KOH. The resin water solution was then put through the juicer again to recover the resin and collect the Bee Product Extract. The pH was adjusted with concentrated sulphuric acid to make it suitable for topical use pH around 5.5 to 7.5. The protein absorbance profile was determined to be similar to that produced from the filtration process. The number of protein particles per mL in the Bee Product Extract was also measured using qNano analysis and shown to be greater than 1 billion particles / mL.

### Example 6: SCX HPLC solid phase synthesis methods

Solid phase synthesis of a Bee Product Extract from Manuka honey was performed by strong cation exchange. While SCX is an analytical method it can be scaled up to a pharmaceutical method. The resin used for SCX HPLC was shown to contain a ligand (in this case p-toluenesulfonate) that appeared to have functionally useful properties for separation of various proteins present within Manuka honey. It is proposed that the separation of the royal jelly protein colloidal particles from Manuka honey was achieved through the use of hydrophobic interaction via pi stacking of aromatic rings present in the phenolic compounds bound to the royal jelly proteins. The Bee Product Extract can be directly applied to the HPLC column and run in water. The binding increased in strength in the presence of a salt, which was counter intuitive with respect to the use of a strong cation exchanger where elution would be typically mediated using salt. Previously, SCX HPLC methods for purification have been considered highly unlikely to be functionally useful in the purification of the royal jelly proteins from Manuka honey. Contrary to this view, a number of different protein complexes were able to be separated using this purification methodology. This novel pi stacking approach to protein recovery from manuka honey was an unexpected finding and is used as the basis for protein purification. It is therefore not ion exchange that is responsible for protein recovery and therefore not infringing on the methods taught in the patent literature and in the scientific literature as to the purification of major royal jelly proteins. This provides a novel and unexpected finding for recovery of proteins from manuka honey.

### PHOTO-FENTON CHEMISTRY and mode of action

Without wishing to be bound by theory, the inventor proposes the therapeutic benefits of the Bee Product Extract are due to the following mode of action.

Figure 14 shows a reaction scheme for Photo-Fenton chemistry breakdown of aromatic rings, and their hydroxylation and generation of a range of small molecule acidic compounds and finally CO₂ and water. The process whereby the hydroxyl radical breaks cells back down into CO2 and water in apoptosis allowing death and regeneration to occur within cells.

### Reaction with hydrogen peroxide

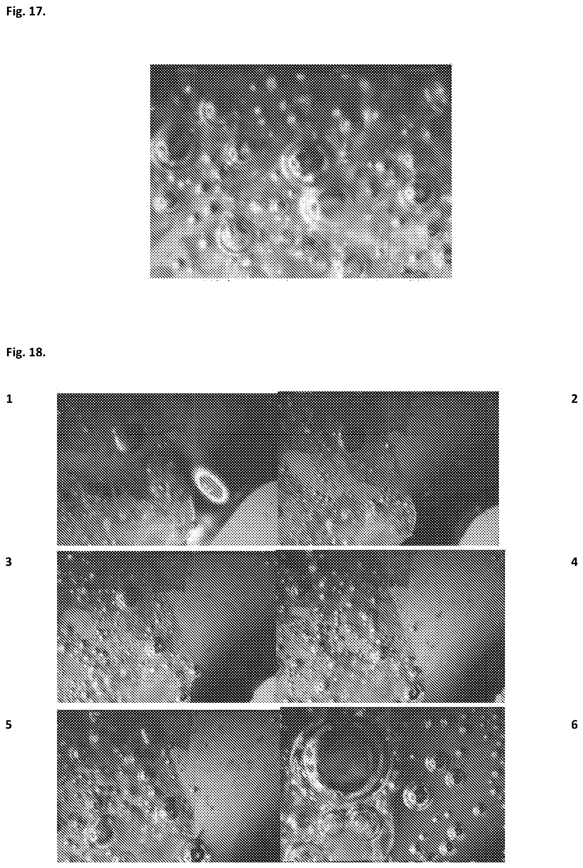

The oxidation reduction of Fe can be accelerated by UVA light. The redox partners DHA and MGO appear to be potential coordination partners with iron. To test if MGO was able to coordinate with Fe(ii) and generate Hydroxyl radicals in the presence of hydrogen peroxide the following materials were put onto a microscope slide and illuminated with UVA light using the DAPI filter cube on the Evos FL microscope. A solution of MGO 40% + Fe(ii)SO₄ + H2O2 2% and phenyllactate (the components of manuka honey present in the Bee Product Extract of the invention) was mixed and illuminated using the UVA LED light on the epifluorescent microscope. Bubbles formed as well as a blue fluorescence within a thin film on the slide. Figure 17 shows at 2 x magnification the bubble formation which occurred under UVA light supplied by a Qdot LED (357 nm). This indicated MGO was able to coordinate with iron and promote photo-fenton type chemistry and the formation of the Hydroxyl radicals and other ROS species such as super oxide could be generated by this simple mixture. The photo-reduction of iron was able to be promoted by UVA light and the coordination of MGO with Fe3+ produced after Fe2+ oxidation.

Figure 18 shows generation of bubbles in Manuka honey when mixed with H₂O₂ 2% solution, shown at 2 x magnification. Bubble formation occurred under UVA light supplied by a Qdot LED (357 nm). This indicates the role of glucose oxidase in the generation of hydrogen peroxide within honey, and the inhibition of glucose oxidase by MGO in preventing fenton chemistry occurring in manuka honey, which provides an opportunity to purify the proteins before fenton chemistry has had a chance to break them down into other molecules.

The identification of photo-fenton chemistry occurring in Manuka honey and the generation of short lived Hydroxyl radial provided a mechanism of action for the antimicrobial properties of Manuka honey. Such a compound is short lived around 1 nanosecond and has 1200 electron volts of energy. The identification of the Hydroxyl radical being the active ingredient within the honey prevents stabilization of such a molecule due to its high reactivity. The lack of glucose oxidase activity means the reaction is prevented or reduced in Manuka honey due to MGO inhibition of glucose oxidase. This novel finding meant that radicals provide energy to the body and promote health and vitality. This is the exact opposite to what the supplement industry claims and therefore is contrary to the current scientific understanding and regarded as an alternative view point.

### Example 7: Apisimin modification during Bee Product Extract production

It is proposed that the Hydroxyls and methyls originate from radicals. Energy is captured by the ring structure and the photo-reduction of the bound mono-atomic mineral provides the reducing potential from light. Fe3+ becomes Fe2+ and this reacts with hydrogen peroxide generating OH radicals. In a light reduction, H2O2 oxidation energy system. It is known that phenolics bind proteins both covalently and non-covalently [15] and such interactions have been identified in honey. The covalent interactions occur when the phenolic becomes oxidised through its action as an anti-oxidant forming quinones and large MW complexes called melanoidins [16]. Recently, it was recognized that honey produces Hydroxyl radicals which are responsible for the anti-microbial activity of honey (Brudzynski and Lannigan, 2012), rather than the presumed hydrogen peroxide content. The production of Hydroxyl radicals has not been previously identified in Manuka honey and as methyl glyoxal content of Manuka honey inhibits glucose oxidase no hydrogen peroxide is produced in Manuka honey and therefore the NPA antibacterial activity is currently unknown. It has been shown that hydrogen peroxide produced in the honey was converted into the Hydroxyl radical and this was enhanced in BuckWheat honey due to the elevated phenolic content [17]. The components needed to produce Hydroxyl radicals are all present in the Manuka honey colloid protein complex which includes the mineral iron and copper, proteins and enzymes (glucose oxidase) and phenolics. However this has not been demonstrated in Manuka honey and is considered novel as this teachers that the active is not MGO, which is currently identified as the active ingredient in the Manuka honey. The inventor therefore considered if the Hydroxyl radical formation was responsible for the bubbling observed or if other ROS species such as superoxide could also have been produced. Fenton chemistry can generate CO2 and H2O through the generation of Hydroxyl radicals and it has been widely used in the remediation of toxins in the environment. The requirement for Fe(ii) or reduced iron is paramount for Fenton chemistry to occur to produce the Hydroxyl radical and other ROS species. The reduction of iron can occur via UVA light, via photon capture by phenolics that coordinate mineral irons such as Fe 3+ via the pi electrons in the aromatic ring of the phenolic structure. This is the proposed mechanism for lithium mode of action binding to pi electrons in the aromatic rings of neurotransmitters such as dopamine and serotonin providing an ability to modulate photo-Fenton chemistry, which occurs in the subconscious mind.

The six-membered ring in benzene is a perfect hexagon (all carbon-carbon bonds have an identical length of 1.40 Å). The area within the benzene ring is 0.051 nm which equates to 0.51 Angstrom. Therefore the smaller lithium atom has a stronger affinity to the benzene ring than sodium due to its smaller size and therefore can displace other elements. The empirical radius for Li is 145 pm, Na 180 pm, K 220 pm, Rb 235 pm, Fe 140 pm, Cu 135 pm and Ca 180 pm. Cation pi interactions have been studied in biology but their involvement in transmutation of minerals has not been identified in biology. The smaller elements iron and copper would also be able to bind and perform photo-reduction.

Increased charge creates a stronger interaction. Therefore the Al3+ has a strongest interaction with a small atomic radius of 125 pm. This may be why Al3+ has considerable toxicity to the brain by strongly binding to the aromatic rings of neurotransmitters. The number of functional coordination groups electron donating for the suppressor neurotransmitters compared to the neurotransmitters that stimulate brain function provides a basis for promoting or inhibiting photo-fenton chemistry of the quantum photonic subconscious mind. The photo-reduction process resulted in the reduction of the bound mineral, so this provided further evidence for our observation of the bubbling of dissolved honey on the microscope when exposed to UVA light. It is known that Fenton chemistry can be enhanced via phenolics due to the ability of the phenolic to form coordination complexes due to the electron donating groups (Hydroxyl and pi electrons of the aromatic ring) with transition metal ions including copper and iron. The high iron content of Manuka honey and the high phenolic content suggested that rather than just Fenton chemistry being responsible for the anti-microbial activity via the production of Hydroxyl radicals in Manuka honey was due to photo-Fenton chemistry. To test this hypothesis the inventor tuned the LEDs to the absorbance profile for the phenolic present in the Manuka honey. When phenyllactic acid was combined with methylglyoxal and Fe(ii)SO4 and hydrogen peroxide bubbles were produced. The reaction rate was increased when viewed under UVA DAPI LED filter cube on the Evos FL epifluorescent microscope. Iron is coordinated to a phenolic or another electron donating compound that is capable of coordinating to iron such as diHydroxylacetone, polymeric glyoxal and methylglyoxal and the capture of photons by the compound bound to iron results in Fe(iii) being reduced to Fe(ii). The reduced Fe(ii) then reacts with hydrogen peroxide generated by active glucose oxidase producing the OH radical and Hydroxyl ion.

Different phenolics appear to be directly linked onto the N-terminus of apisimin. This is triggered by the MALDI TOF laser photon capture and photo-Fenton production of the Hydroxyl radicals. MGO reaction with lysine residues produces radicals. The phenolic adducts are observed. The MSMS data was analyzed and demonstrated to contain the N-terminal fragment of Apisimin (sequence KTSISVKGE). The amino terminal lysine residue at position 1 was not observed which indicated that the linkage between the lysine and the phenolic was most likely to occur at this point. The link between the N-terminal lysine of apisimin and the phenolic was likely to be through a MGO linkage. Some of the masses observed included 1302 Da and 1274 Da. MS/MS analysis of the 1274 Da series material was analysed using MALDI TOF MS/MS. Interestingly only a 1078.2051 Da and a 38.998 Da material was observed. 38 Da could correspond to C=O carbon monoxide. This may be able to turn off oxidative phosphorylation. As iron was present in the sample and the laser produces excess energy the inventor expects the 38 Da material originated from the Fenton reaction. Structure of 1078.2051 M+1 ion (Mass of M = 1077.22936). The mass difference between 1274 and 1077 is 197 Da. Loss of oxygen lead to a mass of 213 Da for the M+1 ion which corresponds to trimethoxybenzoic acid (Mass = 212.2 Da). The UV / Vis spectral profile appears to correspond to methyl syringate. C=O from MGO laser induced breakdown product MS 38 Da. The 1078 Da appeared to correspond to the peptide KTSISVKGES (Apisimin) with MGO attached, which a fragment of the parent ion was corresponding to 1273 Da. Parent ion 1273 Da corresponding to apisimin N-terminus, MGO linked (C3H4 - 40 Da) trimethoxybenzoic acid (MS 212.2 Da) MGO + Trimethoxybenzoic acid (253.2 Da) or methyl syringate (MS 212.2 Da). There are various forms of phenolics in Manuka honey and a number of peaks are observed which are thought to correspond to the various forms of phenolics linked to the end of apisimin through a MGO linkage. MSMS analysis the 1302 Da parent ion was performed. Previously the inventor had isolated a protein of molecular weight 5791.7695 Da, which was 254 Da larger than apisimin 5537.061 Da. 5791-5537 = 254 M+1. , representing a modification of 253 Da. This modification is believed to be that of the structure outlined below.

### Full length modified apisimin

The combination of the phenolic linked through MGO to the N-terminus of Apisimin fragment or full length is suggested as the modified form of apisimin responsible for a range of interesting biological properties. Further analysis revealed other remarkable details about this biomaterial, including its ability to form aggregates of small sizes, its high aliphatic amino acid content making it more lipid like and compatible with human stratum corneum (skin) and its role in the thixotropic properties of manuka honey, its N-terminal end being hydrophilic and containing Serine and glutamate residues as well as the MGO modified lysines. These details point clearly to a completely different hypothesis for the formation of DHA, MGO and energy production within Manuka honey which separates it from other honeys in its unique ability to treat a wide range of human diseases. It also points to a completely new mechanism for energy generation which is driven by primordial evolutionary understanding of the importance of reactive oxygen species in the generation of energy and the damage that highly purified plant based phenolics could be potentially causing due to interference with normal metabolic processes at night that are driven largely due to iron based Fenton reaction radical metabolic energy generation. This is a complete about face on current thinking as to the benefits of anti-oxidants. The information that is outlined below is shown in support of this hypothesis. Parent ion 1273 Da corresponding to Apisimin N-terminus, MGO linked (C3H4 - 40 Da) trimethoxybenzoic acid (MS 212.2 Da) MGO + Trimethoxybenzoic acid (253.2 Da). The intact apisimin with this modification has also previously been identified. Previously a protease in honey has been identified by others however the actual protease has not been identified. One possible reason for this is due to the fact that there is no protease present in the honey and the peptides that are generated over time are due to reactive species generation. The presence of high concentrations of iron which may coordinate to the phenolic compounds present in honey and its potential presence in the pollen in Manuka honey may result in the formation of reactive oxygen species and the generation Fenton reaction to produce OH radical. This radical is able to cleave all compounds due to its high reactivity and it is short lived 10^-9 seconds. It may react with the phenolics that the iron is coordinated to and lead to its breakdown into polymeric glyoxal and then DHA and MGO.

The production of MGO from phenolic compounds via photo-reduction / oxidation cycle provides an alternative understanding to MGO formation within Manuka honey. As mentioned above, MGO modification of lysine residues at the N-terminus of Apisimin prevented coomassie Blue G250 from interacting with the positively charged amino acids residues and therefore staining of apisimin isolated from Manuka honey that contained high concentrations of MGO was not feasible. The absence of apisimin in SDS PAGE gels indicated that the N-terminus was either blocked by MGO modification or buried within the complex structure and not available for Coomassie Blue G250 staining after SDS PAGE analysis.

### Reactive oxygen species

The antioxidant and antibacterial properties of macromolecules in honey are described as forming compact, stable multimeric assemblies (Brudzynski et al., Scientific Reports volume 7, Article number: 7637 (2017). The authors suggest the assemblies are key to the biological activity. The reported absence of catalase in honey is not responsible for the vigorous reaction with hydrogen peroxide. The rate of reaction was accelerated with different wavelengths of electromagnetism. This observation prompted further investigation of what was responsible for the bubbling. The increase in reaction rate as wavelengths of the light used changed from red through to blue and into UVA at 357 nm suggested that light was partly responsible for this observation. It is known that UVA light can split water at pH 1.0 to produce hydrogen and oxygen gas. The pH of the honey was determined to be 4.3. So it was not expected under these conditions that water could be split to form oxygen and hydrogen.

Based on the composition of Manuka honey and the presence of iron, phenolics and hydrogen peroxide produced from glucose oxidase, it is believed that upon dissolving Manuka honey in water on a microscope slide, the reaction of glucose with glucose oxidase results in the formation of hydrogen peroxide. The reactions with hydrogen peroxide are likely to be responsible for a range of redox based reactions that produce reactive oxygen species (ROS) responsible for the bubbling phenomenon observed. The wavelength of light most effective at increasing the bubbling in the dissolved Manuka honey was 357 nm in the UVA region which corresponds to an absorbance maximum of a number of phenolics. The absorbance spectrum of methyl syringate at 350 nm was confirmed indicating its functional features of absorbing light at this wavelength and dependent on pH. It is proposed that the Hydroxyls and methyl groups in methyl syringate originate from radicals. Energy is captured by the ring structure and the photo-reduction of the bound mono-atomic mineral provides the reducing potential from light. Fe³⁺ becomes Fe²⁺ and this reacts with hydrogen peroxide generating OH radicals. In a light-based reduction of iron and the H₂O₂ oxidation energy system, which is damaged in Manuka honey due to MGO inhibition of glucose oxidase. It is known that phenolics bind proteins both covalently and non-covalently and such interactions have been identified in honey. The covalent interactions occur when the phenolic becomes oxidised through its action as an anti-oxidant forming quinones and large MW complexes called melanoidins. Recently, it was recognized that honey produces Hydroxyl radicals which are responsible for the anti-microbial activity of honey [17], rather than the presumed hydrogen peroxide content. The production of Hydroxyl radicals has not been previously identified in Manuka honey and as methyl glyoxal content of Manuka honey inhibits glucose oxidase no hydrogen peroxide is produced in Manuka honey and therefore the NPA anti-bacterial activity is currently unknown but has a correlation to MGO content.

It has been shown that hydrogen peroxide produced in the honey was converted into the Hydroxyl radical and this was enhanced in BuckWheat honey due to the elevated phenolic content. The components needed to produce Hydroxyl radicals are all present in the Manuka honey colloid protein complex which includes the mineral iron and copper, proteins and enzymes (glucose oxidase) and phenolics. However, this has not been demonstrated, is not obvious and as Hydroxyl radicals are considered the most potent of oxidation systems in cells are considered to induce cell death via apoptosis. These findings of the beneficial effects of Hydroxyl radicals in Manuka honey and the Bee Product Extract recovered from Manuka honey is considered novel as this teachers that the active is not MGO, which is currently identified as the active ingredient in the Manuka honey. The inventortherefore considered if the Hydroxyl radical formation was responsible for the bubbling observed as well as other ROS species such as superoxide that has been detected by dye based methods. Fenton chemistry can generate CO₂ and H₂O through the generation of Hydroxyl radicals and it has been widely used in the remediation of toxins in the environment. Therefore, it has the potential to remediate toxins within the human body by breaking them down into CO2 and water. This highly destructure process is occurring in apoptosis and is a natural cellular death process.

The requirement for Fe²⁺or reduced iron is paramount for Fenton chemistry to occur to produce the Hydroxyl radical and other ROS species. The reduction of iron can occur via UVA light, via photon capture by phenolics that coordinate mineral irons such as Fe 3+ via the pi electrons in the aromatic ring of the phenolic structure. This is the proposed mechanism for lithium mode of action binding to pi electrons in the aromatic rings of neurotransmitters such as dopamine and serotonin providing an ability to modulate photo-Fenton chemistry, which occurs in the subconscious mind. The binding of lithium, with its small size and strong binding affinity is expected to displace other minerals that are present in the mind such as iron and calcium and copper and therefore affect the functional role of the subconscious minds physics light based processes reducing the person to an unemotional flattened individual making them easier to control with respect to the highs and lows of bipolar or manic depressive disorder. It is known from the scientific literature that minerals can bind pi electrons in the aromatic ring but this has not at all been connected to memory formation or subconscious mind physics.

The six-membered ring in benzene is a perfect hexagon (all carbon-carbon bonds have an identical length of 1.40 Å). The area within the benzene ring is 0.051 nm which equates to 0.51 Angstrom. Therefore the smaller lithium atom has a stronger affinity to the benzene ring than sodium due to its smaller size and therefore can displace other elements. The empirical radius for Li is 145 pm, Na 180 pm, K 220 pm, Rb 235 pm, Fe 140 pm, Cu 135 pm and Ca 180 pm. Cation pi interactions have been studied in biology but their involvement in transmutation (physics) of minerals has not been identified in biology. The smaller elements iron and copper would also be able to bind and perform photo-reduction reactions. Increased charge creates a stronger interaction. Therefore, the Al3+ has a strongest interaction with a small atomic radius of 125 pm. This may be why Al3+ is highly toxicity to the brain by strongly binding to the aromatic rings of neurotransmitters preventing the appropriate minerals from binding and the proper structures and quantum tunneling of hydrogen cannot occur so the memory system is somewhat blocked by Al3+ due to its binding strength. The photo-reduction process resulted in the reduction of the bound mineral, so this provided further evidence for our observation of the bubbling of dissolved honey on the microscope when exposed to UVA light. It is known that Fenton chemistry can be enhanced via phenolics due to the ability of the phenolic to form coordination complexes due to the electron donating groups (Hydroxyl and pi electrons of the aromatic ring) with transition metal ions including copper and iron. The high iron content of Manuka honey and the high phenolic content suggested that rather than just Fenton chemistry being responsible for the anti-microbial activity via the production of Hydroxyl radicals in Manuka honey was due to photo-Fenton chemistry. To test this hypothesis the inventor tuned the LEDs to the absorbance profile for the phenolic present in the Manuka honey. When phenyllactic acid was combined with methylglyoxal and Fe(ii)SO4 and hydrogen peroxide bubbles were produced. The reaction rate was increased when viewed under UVA DAPI LED filter cube on the Evos FL epifluorescent microscope. The following scheme is proposed, whereby iron is coordinated to a phenolic or another electron donating compound that is capable of coordinating to iron such as diHydroxylacetone, polymeric glyoxal and methylglyoxal and the capture of photons by the compound bound to iron results in Fe(iii) being reduced to Fe(ii). The reduced Fe(ii) then reacts with hydrogen peroxide generated by active glucose oxidase producing the OH radical and Hydroxyl ion. As MGO inhibits glucose oxidase in Manuka honey the generation of hydrogen peroxide is impeded. This stops the system at a point where the monoatomic iron is photo-reduced as Fe2+ awaiting reaction with H2O2 in the body of a person using the topical spray. The position of the phenolic-mineral complex appears to be ideal for this reduced iron delivery system that can be used through the skin of an individual for the purpose of supporting the cellular death and regeneration system enabling vitality and health to be restored as well as reducing pain and supporting mental well-being.

The antiviral benefits of honey and bee products is highly relevant in the time of the COVID-19 pandemic. As the inventor has developed an active ingredient form of Manuka honey that is bioavailable through inhalation as it has shown suitable benefits in allergies. The inhaled form of the Manuka honey active ingredient is expected to have considerable benefits in treatment of individuals with bacterials and viral and fungal and yeast infections. Understanding these benefits and the novel formulation provides a step change in the fight against antibiotic resistance and this has been a major driver in the transition back to natural alternative to antibiotics because of the inability of bacteria and viruses to develop resistance to the Hydroxyl radical. It is therefore expected based on the mode of action identified that the topical Manuka honey spray will be ideal in wound and burn treatments as well as an inhaled anti-allergic and anti-viral formulation of Manuka honey that can be used in a non-contact way.

Previously a protease in honey has been detected by others, however the actual protease has not been identified. One possible reason for this is due to the fact that there is no protease present in the honey and the peptides that are generated over time are due to reactive species generation. The presence of high concentrations of iron which may coordinate to the phenolic compounds present in honey and its potential presence in the pollen in Manuka honey may result in the formation of reactive oxygen species and the generation Fenton reaction to produce OH radical. This radical is able to cleave all compounds due to its high reactivity and it is short lived 10⁻⁹ seconds. It may react with the phenolic that the iron is coordinated to and lead to its breakdown into DHA and MGO.

The Hydroxyl radical can then react with other species that act as anti oxidants the phenolics which triggers the production of methyl syringate from other phenolics within the honey. The inventor has also investigated exposing water diluted Manuka honey to UV light and have seen an increased concentration of methyl syringate after UV exposure. This indicates that the Hydroxyl radical reacts with the antioxidant phenolics to produce methyl syringate. The exposure of diluted Manuka honey to UVA results in changes in the composition of the honey. The generation of superoxide anion has been detected with nitrotetrazolium blue (NTB).

### NTB reaction with Manuka honey demonstrating the production of superoxide radicals

When Manuka honey is exposed to UV light in the presence of NTB, a compound that reacts with superoxide to form a precipitate, a reaction occurs and a purple precipitate is generated on the microscope slide. The exposure to light (UV) high energy appears to cause the formation of reactive oxygen species in Manuka honey leading to its aging. Changes in the chemical composition of the honey proteins after exposure to UV light are expected and this may result in the formation of adducts between phenolics and proteins. When Manuka honey was dissolved in water and NTB the superoxide radical scavenger was added to the solution the formation of a purple precipitate was detected. The use of UV light of Ex 357±44, Em 447±60 additional superoxide was generated as the colour changed more rapidly. Manuka honeys were able to do this at a faster rate than clover honey.

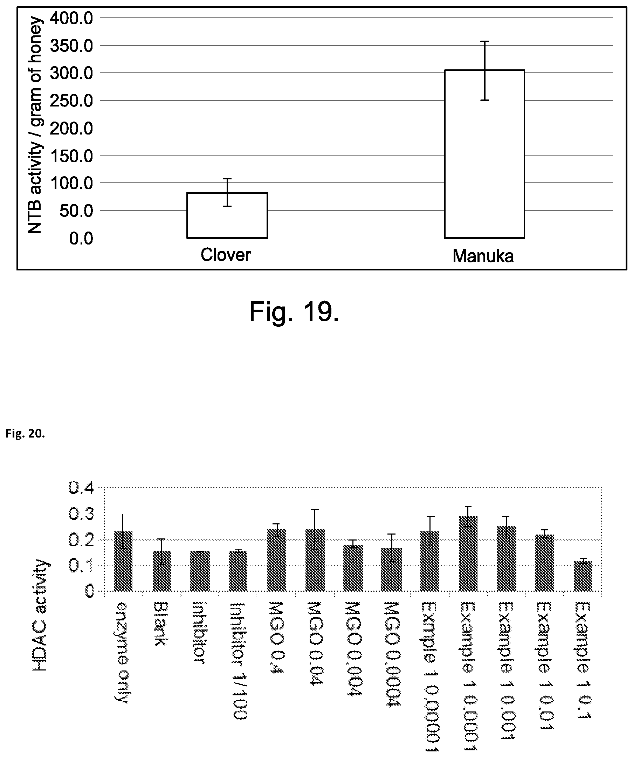

Figure 19 shows NTB activity of a manuka honey, and clover honey. Both honeys were dissolved in DMSO containing NTB and then reacted in the presence of UVA light. The amount of superoxide radical generated by the manuka honey was greater than that of clover honey due to differences in the composition of the honeys given the higher phenolic and mineral content of manuka honey.

The photo-reduction chemistry lead to rapid production of Hydroxyl radicals that connect the compounds together and generate the unique molecular structures within the MALDI TOF Ms instrument and it is proposed that similar reactions occur within cells generating phenolic-MGO-apisimin peptides adducts in the phagosome due to the reaction with chemical energy of hydrogen peroxide. The products from the MS analysis reveal the close proximity of the N-terminus of apisimin within the cavity of the colloidal complex of MRJP1-apisimin-24 methylene cholesterol which has x-ray structural data (without the N-terminus). The phenolics present in Manuka honey would appear to be held within the apisimin-MRJP1 complex and monoatomic iron associated with the phenolic ring pi electrons. This enclosed environment when exposed to the MALDI TOF MS laser generated N-terminal truncated adducts.

The main components of Manuka honey were investigated and the combination of MGO, reduced iron and hydrogen peroxide reacted together rapidly to form bubbles. Adding phenolics into this mixture and using blue or UVA light increased the rate of reaction. The identification of photo-fenton chemistry occurring in Manuka honey and the generation of short lived Hydroxyl radicals provided a mechanism of action for the antimicrobial properties of Manuka honey. Such a compound is short lived around 1 nanosecond and has 1200 electron volts of energy released which turns biological material back into CO2 and water. The identification of the Hydroxyl radical being the active ingredient within Manuka honey prevents stabilization of such a molecule due to its high reactivity. The lack of glucose oxidase activity means the reaction is prevented or reduced in Manuka honey due to MGO inhibition of glucose oxidase. This novel finding meant that radicals provide energy to the body and promote health and vitality in a topical form. This is the exact opposite to what the supplement industry claims and therefore is contrary to the current scientific understanding and regarded as an alternative view point. Therefor the art teaches away from this thinking and it is therefore considered highly novel and a revolutionary approach to health.

Removal of Bee Product Extract from honey has been used to improve the aesthetic and consumer pleasing properties of honey. Manuka honey is very expensive and receives a premium in the market because of the research backing the benefits of this product. The proteins in honey have been shown to form colloidal particles in the micron size but upon dilution in water their size decreases into nanoparticles. This structural rearrangement can be attributed to changes in polarity, and changes in aggregation also occur due to pH. The presence of histidine residues on MRJP1 means that under acidic conditions the His residues are protonated giving a positive charge allowing electrostatic interactions to occur and aggregation.

The dissolving of honey in water activates glucose oxidase allowing hydrogen peroxide formation which generates Hydroxyl radicals from reduced iron and copper present in the honey. The radical reactions generate a wide range of chemicals and break and create a number of bonds. These uncontrolled radical cascades are considered detrimental by the supplement industry and the widespread use of antioxidants are marketed with health giving properties currently. However, as Manuka honey contains MGO, which reacts with and inhibits glucose oxidase, hydrogen peroxide is not produced and therefore the generation of Hydroxyl radicals are not formed as a consequence the proteins are not broken down and the system is relatively stable and can be isolated as described in the specification leading to many benefits in human and animal health.

The addition of water increases the reactivity of methylgloxal towards Arg, Lys and Cys residues which generates radicals. The degree and complexity of the reactions means little if any control over the composition of the material occurs. Therefore batch to batch variability is apparent. Speed of processing is therefore of considerable importance and the separation of glucose from the Bee Product Extract enables a stabilized product if any glucose oxidase activity remains in the honey. The approach developed is rapid and can be used to recover the targeted bioactives in a simple cost effect way.

### Example 8: Pain relief properties

Endocytosis pathways can be subdivided into four categories: namely, receptor-mediated endocytosis (also known as clathrin-mediated endocytosis), caveolae, pinocytosis, and phagocytosis. Clathrin has been implicated as a necessary component of phagocytosis. Clathrin-coated pits are found in peritoneal macrophages, and are located at surface adhesion sites and phagosomes in the macrophage. Phagocytosis is a clathrin dependent process. Pitstop 2 is a potent inhibitor of clathrin-independent endocytosis. The inhibition of uptake of the royal jelly proteins from the Bee Product Extract of Example 1, indicates that uptake into cells is a clathrin independent process and therefore not phagocytosis, which clearly provides an understanding for the suitability of the formulation technology based on Bee Product Extract to be taken up into a wide range of cells therefore providing a source of protein, mineral, and phenolic as well as other pharmaceutical ingredients that can be employed to deliver health promoting benefits to humans and animals. The delivery of vitamins and minerals can be employed using this cellular delivery formulation providing a protective protein cage so that the ingredients within the protein cage can be maintained intact for internal cellular delivery.

The pain free healing technology can be used to improve patient compliance as dressing changes are not going to be painful as the Hydroxyl radical causes a switching off of pain signals from macrophage as part of the phagocytosis inhibition. The chemical reactivity causes a wide range of additional products to be produced when entering into the cell through phagocytosis as the exposure of the reduced iron to hydrogen peroxide occurs as NADPH oxidase releases hydrogen peroxide in the phagosome. The array of molecules produced is dependent on the phenolic-mineral-protein complex under a set of circumstance that is highly complex. Due to the complex nature of the product and the reactivity of OH* with a wide range of bonds it is likely the formation of phenolic-mineral-protein complexes occur as part of the radical cascade chain termination events due to the known anti-oxidant nature of the phenolic species in Manuka honey. Individual species are therefore somewhat irrelevant as it is the process of OH* generation in the latency of Fe2+ light reduction and location of hydrogen peroxide reactivity that is responsible for the widespread range of clinically observed benefits obtain from the product.

### Example 9: HDAC inhibition by Bee Product Extract of Example 1

Confirmation of HDAC inhibition using Epigenase HDAC Activity/Inhibition Direct Assay Kit (Colorimetric) 96 Assays Cat # P-4034-96 and HDAC1 as the purified enzyme was performed. Epigenase HDAC Activity/Inhibition Direct Assay Kit (Colorimetric) 96 Assays Cat # P-4034-96 (https://www.epigentek.com/docs/P-4034.pdf) was used to confirm the HDAC inhibition targeting HDAC1 (Huntingtree Associates Ltd) of the Bee Product Extract prepared from manuka honey. A positive and negative control was used along with the Bee Product Extract produced in Example 1. This assay system is sensitive and does not suffer from fluorescent quenching issues that potentially plague the current assay performed (Enzo - FLUOR DE LYS-Green HDAC fluorometric activity assay kit) due to the known fluorescent compounds present in Bee Product Extract. HeLa cell nuclear preparation containing multiple HDAC enzymes was also used to identify HDAC inhibition. This extract potentially contains both Class I and II HDACs whereas HDAC1 is a Class I HDAC and a suitable target for immune-modulatory activity for IBS [18] as well as anticancer and other anti-inflammatory conditions.

MGO modified lysine residues occurs within proteins in the Bee Product Extract produced from manuka honey. MGO in Manuka honey produces the adduct N(epsilon)-(carboxyethyl)lysine (CEL). The CEL adduct may have activity as an inhibitor towards HDACs and therefore have potential anti-cancer properties via HDAC inhibition. The analysis of HDAC inhibition of Manuka honey Bee Product Extract was tested using the bioassay. Activity as an inhibitor towards HDACs and therefore the potential anti-cancer properties and other anti-inflammatory properties for the treatment of other conditions such as IBD could be evident. The analysis of HDAC inhibition of both MGO and the Bee Product Extract of Example 1 was determined as shown in Figure 20, and shows a dose response inhibition of HDAC. Student Ttest was performed and shown to have 95% confidence that the Bee Product Extract was inhibitory toward HDAC enzyme tested in this assay (p < 0.048) at a 0.1% (10 times) dilution of the original product of Example 1.

### Example 10: Mental health; bipolar disorder

There are no effective animal models for the brain health stimulatory benefits in humans, as all animal models have comparative difference to the human being and the human mind. The Bee Product Extract produced from manuka honey prepared using Example 1 was evaluated in a number of clinical case studies for improvements in brain health in patients in the following areas of brain health including bipolar disorder, Alzheimer's disease, anxiety, depression, brain fog and increased cognition and learning ability.

Patient X diagnosed with bipolar disorder in 2013, was placed on lithium and then stopped due to obesity issues had another hypomanic episode was placed on respiradone and valproate for several years. The individual then started using Bee Product Extract of Example 1 as a topical spray and was able to reduce medication and is now living independently and able to conduct his business affairs without medication and was shown to be both healthy and happy without bipolar swings in mood through the managed use of the Bee Product Extract as a topical spray.

### Example 11: Reduction of allergenic properties due to MGO modification of MRJP proteins

The presence of MGO in Manuka honey allows the modification of Lys, Arg, and Cys residues in complex ways. The structure of DHA in honey with its low water environment is in a dimer, where it is unable to form MGO because of the DHA dimer is unable to react to form MGO only the monomer can undergo this rearrangement. This means the approach that has been taken to prepare the Bee Product Extract provides the necessary reactivity to generate MGO from DHA as the addition of water provides the needed chemistry for DHA to MGO conversion. The modifications of Lysine, Arginine and Cys by MGO allows for the generation of additional ingredients. Lysine adducts have been demonstrated to inhibit HDAC and the lysines within the allergenic epitopes of the Bee Product Extract when reacted with MGO under these conditions have been shown to no longer convey allergies in those who have previously had allergenic reactions with bee proteins. This again confirms the beneficial properties of the product with respect to reducing the potential allergenic properties of the Bee Product Extract using the methods outlined in this specification.

It is known that some people have allergies to bee proteins and a range of MRJP protein sequences have been identified as being allergenic. All of the peptides with allergenic properties contain lysine residues. Others have shown in the art that chemical modification of these lysine residues can therefore modify the allergenic properties of the MRJP proteins. The method by which MRJP proteins are isolated from Manuka honey provides ample opportunity for the lysine residues in the MRJP proteins to be modified by MGO leading to CEL motifs, that not only provides a dampening down of any potential allergenic property of the MRJPs purified but also provides a HDAC inhibitory property to the protein giving it a therapeutic benefit in modulating HDAC enzymes which makes the proteins have additional benefits towards cancer prevention and reduction of inflammation. HDAC inhibitors induce cancer cell cycle arrest, differentiation and cell death. Moreover, they reduce angiogenesis and modulate immune response. Hypothesis of "epigenetic vulnerability of cancer cells", which has been proposed (Dawson MA, Kouzarides T. Cancer epigenetics: from mechanism to therapy. Cell. 2012 Jul 6; 150(1):12-27), is a cause of relative specificity of HDAC inhibitors. Histone deacetylase (HDAC) inhibitors have been developed clinically for malignancies due to their effects on apoptosis. More recently, in vitro and in vivo data indicates that HDAC inhibitors may be anti-inflammatory due to their effects on cell death acting through acetylation of non-histone proteins. Along those preventative properties and therapeutic benefits of the MRJPs purified from Manuka honey anticancer and anti-inflammatory properties are highly evident and of considerable economic benefit because of the issues around auto immune disease, organ transplantation, toxic shock and the cytokine storm that occurs in autoimmune disease as well as in the mRNA mock vaccine therapies being developed for COVID-19 prevention.

The MPRO enzyme in SARS-CoV-2 is a thiol enzyme that is susceptible to oxidation and therefore inhibition by the Hydroxyl radical that is generated in the royal jelly Bee Product Extract from manuka honey through the light based photo-reduction of iron bound to the aromatic ring of the phenolics present in manuka honey which are bound to the Bee Product Extract. By isolating the proteins the Hydroxyl radical can be used to promote health and well-being, disease prevention and regeneration through supporting the apoptosis process. Inhibition of the cytokine storm in COVID-19 treatment, its inhalation formulation and demonstration of its safety in the use for hay fever treatment means that the product prepared from manuka honey is able to support the health and well-being of people who have health issues including COVID-19 and other bacterial and viral diseases.

With the rise of antibiotic resistance it is of considerable importance to be able to develop new and effective treatments for a wide range of human diseases due to pathogens and their ability to cause illness. TB is an example of a human disease where multiple drug resistance has been observed. The failure of antibiotics means treatments are no longer possible. The use of the royal jelly Bee Product Extract isolated from manuka honey with its known antimicrobial and antiviral properties is therefore seen as a viable alternative with a long history of safe and effective use.

The generation of the Hydroxyl radical (OH*), with its 1200 electron volts (eV) released in 1 nanosecond has the ability to react with all biological chemicals in the body and convert these into CO2 and water, which is the basis for apoptosis. ATP is no longer available to do work in the cell as the mitochondria are not functional during apoptosis. Apoptosis or pre-programmed cell death generates apoptotic bodies that are released from the parent cell and are taken up by other cells around the body. The Hydroxyl radical produces a quantum entangled fluid in the apoptotic bodies, which enables a quantum based communication system, or spooky action at a distance throughout the body, that enables cellular communication at a distance and it is the most fundamental communication system within the human body that the whole organism works as one rather than 50 trillion individual cells.

It is known that radicals are damaging to cells. It is known that radicals cause cascades. But it is not known that radicals are an early energy system within the body that provides a widespread quantum communication system that ensure collectivism within the body. As the radical jumps from one molecule to another it acts as a thread bringing all of those molecules together ensuring that they are able to respond in an appropriate way to the energy within the local environment. This system is the fluid within the apoptotic bodies and is taken up by other cells. Once in the cell it provides a direct link to what has previously happened in the parent cell prior to apoptosis. This communication system is seen in eVs (extracellular vesicles) which is a new area exciting area of scientific investigation.

The quantum nature of this fundamental communication system driven by Hydroxyl radicals puts a new perspective to the role of radicals in our bodies and provides a context for the detrimental role of anti-oxidants are playing in the body interfering with this deeper communication system that enables the body to continue to work as one in a holistic manner. The outcome of this understanding is to teach away from the current art and the narrative of benefits that antioxidants have in our bodies preventing disease. The clinical case studies and literature evidence support the claims being made in relation to the support of cell death (apoptosis) by the MRJP protein complexes purified out of Manuka honey and the regenerative benefits of such an approach. Removing old misfolded proteins using such a technique has been shown to have a wide range of clinical benefits including mental well-being and diseases of the mind, pain relief and wound healing, anti-bacterial properties, antiviral properties and anti-allergic properties, and anti-inflammatory properties. It therefore, appears that adding radicals to the human body increases vitality.

As apoptosis occurs within 50 billion cells in our bodies every day and is a necessary process, our biological sensors do not detect our cells dying within our bodies. The pain free nature of the health benefits (healing) of the product produced in this specification is produced by the process developed also relates to an oxidative switch that turns off pain [19]. The OH* generated using photo-Fenton chemistry performs that switch. It also turns off macrophage cells from sending pain signals that are involved in pro-inflammatory processes within the body. Turning off macrophage signaling by inhibition of endocytosis is also important in stopping pain in its tracks. Therefore, the product has remarkably demonstrated a clinical benefit of pain relief within seconds of use in humans in clinical case studies through the external spraying of the product leading to healthier and happier people. Those benefits have been observed in a number of people as outlined in the examples (clinical case studies) to demonstrate the health benefits of the product produced exemplifying the technology developed and clearly demonstrating the clinical benefits exemplifying the therapeutic benefits produced.

The method developed produces a cost effective product that contains MRJP proteins recovered in a way that retains their biological complexity to produce OH* and this is able to be delivered topically in order to produce a therapeutic benefit to people and animals for a wide range of human conditions, illnesses and diseases both chronic and acute allowing functional health to be restored. In addition, the product provides the ability to prevent disease through supporting the innate immune systems ability to fight disease and support the quantum communication system that occurs within our bodies through the apoptosis process supported by Hydroxyl radicals generated by photo-Fenton chemistry.

Royal jelly proteins extracts are commercially available but again these are produced by recovery of the MRJP proteins from queen cells in the hive and the labour intensive method of recovery makes it an expensive product. The proteins are also in a gel like state and usually frozen or freeze-dried is needed to generate a stable product that is used orally as a supplement. The proteins from the hive are in a concentrated complex gel structure and due to the pH are not in a nanoparticle form, which is only obtained after changing the pH of the solution from acidic to basic. This knowledge was combined with the ion-exchange binding knowledge to produce a simple process that has the least number of steps to minimize both costs and ensure the complexes are retained in an active form for topical delivery through the skin due to the size and due to the light based generation of OH* via photo-Fenton chemistry. There are a wide range of health benefits attributed to both Manuka honey, honey in general and to royal jelly proteins and other bee derived products such as propolis or the bees resin materials, including pollen and bee venom. All of these products are taken orally, some in cosmetics and stings used therapeutically in some conditions for the treatment of pain due to phospholipase based inhibition or alternatively the modulation of ion channel function.

Cellular uptake of protein particles (Bee Product Extract) enables the delivery of the proteins (containing the photo-reduced iron (Fe2+) into the cells macrophage which then exposes the Fe2+ to hydrogen peroxide produced by NADPH oxidase. This results in the generation of Hydroxyl radicals and completes the photo-fenton chemical reaction within the phagosome and endosome. Pitstop 2 inhibition identifies that clathrin independent endocytosis is the mechanism by which the Bee Product Extract particles are taken up into the cells. This means uptake is not limited to the inflammatory cells and phagocytosis events which have been outlined as having been induced by mannose 6 phosphate receptor mediated endocytosis and RAGE receptors as Arg-MGO reactions provide a receptor mediated interaction for cellular uptake.

The processes outlined herein delivers light energy into the cells as bonds are broken through the radical cascade process. Where each bond broken corresponds to a wavelength of electromagnetism (positron / electron pairing = photon). The light released produces energy within the body to do work within the cell. It is both a form of eV (electron volts) that supports cellular health and regeneration.

The radical cascades also induce quantum entanglement into the apoptotic bodies and restores the cellular communication system that occur via extracellular vesicles (apoptotic bodies), to maintain coordination of cellular health throughout the body. This provides anti-cancer properties as the hydroxyl radical supports apoptosis and results in the removal of old cells from the body supporting health and well-being preventing disease.

The novel approach of delivery (topical) of the discovered light based (photo-Fenton chemistry) in Manuka honey being responsible for the generation of Hydroxyl radicals and the role of such high energy electrons in the human body (part of apoptosis) means a radically different approach which is outside of the current paradigm of science has been developed to deliver a product that has both marketable benefits in human health and well-being as well as radically different approach to manufacturing methods. Such a process has been developed in order to enable the production of a topical spray that can deliver therapeutic benefits to a human or animal or plants for the purpose to improving health and well-being, vitality and mental health. These novel and unexpected findings make the approach very different from the art being taught currently in science for protein purification at an industrial scale as well as at a laboratory scale and the use of such a light based photo-reduction system in the generation of radicals and therefore beneficial properties in human for health purposes is also a complete 180 degree.

Hydroxyl radicals are considered the most damaging of all the oxidants in cell biology and can destroy all biological structures resulting in cell death. The health and well-being community has for the past 60 years made claims around the use of antioxidants for improving people's health and well-being. This is now being disputed by these findings and the clinical evidence obtained through the use of Bee Product Extract, and an understanding of the mode of action of photo-Fenton chemistry and the novel discovery of a topical spray formation that supports regeneration using the Bee Product Extract, that is natural without additional preservatives.

### INDUSTRIAL APPLICABILITY

As is evident from the above, the present invention is based on an original knowledge that Bee Product Extract of the invention whose preparation using solid phase isolation manufacturing methods creates a water soluble colloidal protein material that interacts with light (electromagnetism) to produce the Hydroxyl radical through photo-Fenton chemistry and the product once recovered shows remarkable bioactivity and keeps the useful pharmacological action of royal jelly proteins, MGO and phenolic mineral complexes. Since the Bee Product Extract has not shown allergenic properties, it can be used by mammals including human easily and comfortably as a topical spray for keeping and increasing their health, reducing pain and accelerating healing of the mind, body and Soul. The Bee Product Extract of the present invention, having the above merits, can be advantageously used as various compositions such as foods, beverages, feeds, pet foods, and cosmetics by incorporating with other ingredients or alone and has demonstrated both pharmaceutical and medical device benefits. The Bee Product Extract has also been shown to be an effective formulation topical, oral and respiratory delivery vehicle of a range of phenolics and minerals and provides a natural way of producing health in an individual in need of such a product. The Bee Product Extract has also demonstrated remarkable benefits to human subconscious mind biochemistry based on monoatomic minerals and the physics of these single atoms, their properties and role in creating memory-boosting benefits in the mind of the individual who are using such products. The Bee Product Extract has shown remarkable ability to perform quantum entanglement and as such can be used to create quantum entanglement in fluids and therefore support the communication in biology connected with apoptosis bodies and the cellular death and regeneration system thereby reducing the accumulation of aging in individuals. The Bee Product Extract has also been noted by individuals who have used the product to support increased fertility and vitality.

It is to be understood that, if any prior art publication is referred to herein, such reference does not constitute an admission that the publication forms a part of the common general knowledge in the art, in New Zealand or any other country.

In the claims which follow and in the preceding description of the invention, except where the context requires otherwise due to express language or necessary implication, the word "comprise" or variations such as "comprises" or "comprising" is used in an inclusive sense, i.e. to specify the presence of the stated feature but not to preclude the presence or addition of further features in various embodiments of the invention.

### REFERENCES

[1] Majtan J, Bohova J, Prochazka E, Klaudiny J. Methylglyoxal may affect hydrogen peroxide accumulation in manuka honey through the inhibition of glucose oxidase. J Med Food. 2014 Feb;17(2):290-3. doi: 10.1089/jmf.2012.0201. Epub 2013 Nov 5. PMID: 24192110; PMCID: PMC3929242.
[2] https://www.mpi.govt.nz/food-business/honey-bee-products-processing-requirements/manuka-honey-testing/
[3] Brudzynski K, Lannigan R. Mechanism of Honey Bacteriostatic Action Against MRSA and VRE Involves Hydroxyl Radicals Generated from Honey's Hydrogen Peroxide. Front Microbiol. 2012 Feb 7;3:36. doi: 10.3389/fmicb.2012.00036. PMID: 22347223; PMCID: PMC3273858.
[4] Nakayama M, Saito K, Sato E, Nakayama K, Terawaki H, Ito S, Kohno M. Radical generation by the non-enzymatic reaction of methylglyoxal and hydrogen peroxide. Redox Rep. 2007;12(3):125-33. doi: 10.1179/135100007X200182. PMID: 17623519.
[5] AU2013277858A1 - Jaganmohan BILLAKANTI, Ulrike Hubl, Keryn Johnson, Cynthia SUN
[6] Kohno K, Okamoto I, Sano O, Arai N, Iwaki K, Ikeda M, Kurimoto M. Royal jelly inhibits the production of proinflammatory cytokines by activated macrophages. Biosci Biotechnol Biochem. 2004 Jan;68(1):138-45. doi: 10.1271/bbb.68.138. PMID: 14745176.
[7] Xueyu Wang, Jie Dong, Jiangtao Qiao, Gensheng Zhang & Hongcheng Zhang (2020) Purification and characteristics of individual major royal jelly protein 1-3, Journal of Apicultural Research, 59:5, 1049-1060
[8] Tian, W., Li, M., Guo, H. et al. Architecture of the native major royal jelly protein 1 oligomer. Nat Commun 9, 3373 (2018). https://doi.org/10.1038/s41467-018-05619-1
[9] Jonathan W. White Jr. & Orest N. Rudyj (1978) The Protein Content of Honey, Journal of Apicultural Research, 17:4, 234-238
[10] Chua LS, Lee JY, Chan GF. Honey protein extraction and determination by mass spectrometry. Anal Bioanal Chem. 2013 Apr;405(10):3063-74. doi: 10.1007/s00216-012-6630-2. Epub 2013 Jan 8. PMID: 23292042.
[11] Tian W, Li M, Guo H, Peng W, Xue X, Hu Y, Liu Y, Zhao Y, Fang X, Wang K, Li X, Tong Y, Conlon MA, Wu W, Ren F, Chen Z. Architecture of the native major royal jelly protein 1 oligomer. Nat Commun. 2018 Aug 22;9(1):3373. doi: 10.1038/s41467-018-05619-1. PMID: 30135511; PMCID: PMC6105727.
[12] Kucharski, R.; Maleszka, J.; Foret, S.; Maleszka, R. (13 March 2008). "Nutritional Control of Reproductive Status in Honeybees via DNA Methylation". Science. 319(5871): 1827-1830.
[13] Leung, R; Ho, A; Chan, J; Choy, D; Lai, CK (March 1997). "Royal jelly consumption and hypersensitivity in the community". Clin. Exp. Allergy. 27 (3): 333-6.
[14] Lombardi C, Senna GE, Gatti B, Feligioni M, Riva G, Bonadonna P, Dama AR, Canonica GW, Passalacqua G (1998). "Allergic reactions to honey and royal jelly and their relationship with sensitization to compositae". Allergol. Immunopathol. 26(6): 288-290.
[15] S 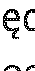 czyk 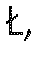 , Świeca M, Kapusta I, Gawlik-Dziki U. Protein Phenolic Interactions as a Factor Affecting the Physicochemical Properties of White Bean Proteins. *Molecules.* 2019;24(3):408. Published 2019 Jan 23. doi:10.3390/molecules24030408
[16] Martinez-Gomez A, Caballero I, Blanco CA. Phenols and Melanoidins as Natural Antioxidants in Beer. Structure, Reactivity and Antioxidant Activity. Biomolecules. 2020;10(3):400. Published 2020 Mar 4. doi:10.3390/biom10030400
[17] Brudzynski K, Abubaker K, Wang T. Powerful bacterial killing by buckwheat honeys is concentration-dependent, involves complete DNA degradation and requires hydrogen peroxide. Front Microbiol. 2012;3:242. Published 2012 Jul 4. doi:10.3389/fmicb.2012.00242
[18] Glauben R, Siegmund B. Inhibition of histone deacetylases in inflammatory bowel diseases. Mol Med. 2011;17(5-6):426-433. doi:10.2119/molmed.2011.00069
[19] Barry CM, Matusica D, Haberberger RV. Emerging Evidence of Macrophage Contribution to Hyperinnervation and Nociceptor Sensitization in Vulvodynia. Front Mol Neurosci. 2019;12:186. Published 2019 Aug 6. doi:10.3389/fnmol.2019.00186

## Claims

1. A batch method for processing manuka honey to form a bee product extract, the method comprising:
a) Combining the honey with water and a cation exchange resin comprising a sulphur propyl or p-toluenesulfonyl ligand and a cellulose or agarose matrix;
b) Adjusting the pH of the mixture to a pH in the range of 2.5 - 3.0;
c) Filtering the mixture to form a solid, and optionally drying the solid;
d) Mixing the solid with water;
e) Adjusting the pH of the mixture to between 8.0 and 10.5;
f) Filtering the mixture and recovering the filtrate; and
g) Optionally, repeating steps b) to f).

2. The method according to claim 1, wherein the cation exchange resin is a SP polymeric material in a solid form.

3. The method according to claim 2, wherein the resin is an agarose-based SP resin such as SP Big Beads, or SP cellulose of a sufficient particle size to enable filtration and pressure based methods to recover the filtrate from the solid.

4. The method according to claim 2 or 3, wherein the filtration steps c) and f) are performed by a cloth filter, filter press or RVF filtration, preferably by filter press.

5. The method according to any one of claims 2 to 4, where the solid obtained in step c) is washed with water at a low pH to decrease the sugar content and retain the protein bound on to the resin.

6. The method according to any one of claims 2 to 4, where the solid obtained in step c) is filtered and pressed without washing, to remove as much sugar solution as possible before step d).

7. The method according to any one of claims 1 to 6, wherein the mixture is maintained at step (c) for a long contact time, e.g. hours, days, weeks or months, to allow protein binding and/or to allow for germination of pollen present in the honey.

8. The method of any one of claims 1 to 7, wherein the resin is separated from the liquid phase by centrifugation.

9. The method of any one of claims 1 to 8, wherein the cation exchange medium is SP cellulose HG2 resin.

10. A Bee Product Extract obtained or obtainable by the method of any one of claims 1 to 9.

11. A pharmaceutical composition comprising a Bee Product extract according to claim 10.

12. A pharmaceutical composition according to claim 11, wherein the composition is formulated as a topical or sublingual preparation.

## Patentansprüche

1. Chargenverfahren zum Verarbeiten von Manuka-Honig zu einem Bienenproduktextrakt, wobei das Verfahren Folgendes umfasst:
a) Kombinieren des Honigs mit Wasser und einem Kationenaustauschharz, das einen Schwefelpropyl- oder p-Toluolsulfonyl-Liganden und eine Cellulose- oder Agarose-Matrix umfasst;
b) Einstellen des pH des Gemischs auf einen pH in der Spanne von 2,5-3,0;
c) Filtern des Gemischs, um einen Feststoff zu bilden, und optional Trocknen des Feststoffes;
d) Mischen des Feststoffes mit Wasser;
e) Einstellen des pH des Gemischs auf zwischen 8,0 und 10,5;
f) Filtern des Gemischs und Gewinnen des Filtrats; und
g) optional Wiederholen von Schritt b) bis f).

2. Verfahren gemäß Anspruch 1, wobei das Kationenaustauschharz ein polymeres SP-Material in einer festen Form ist.

3. Verfahren gemäß Anspruch 2, wobei das Harz ein agarosebasiertes SP-Harz wie SP Big Beads oder SP-Cellulose einer ausreichenden Partikelgröße ist, um filtrations- und druckbasierte Verfahren zu ermöglichen, um das Filtrat aus dem Feststoff zu gewinnen.

4. Verfahren gemäß Anspruch 2 oder 3, wobei die Filtrationsschritte c) und f) durch einen Gewebefilter, Filterpresse oder RVF-Filtration, bevorzugt durch Filterpresse, durchgeführt werden.

5. Verfahren gemäß einem der Ansprüche 2 bis 4, wobei der Feststoff, der in Schritt c) erhalten wird, mit Wasser bei einem niedrigen pH gewaschen wird, um den Zuckergehalt zu verringern und das Protein, das an das Harz gebunden ist, zurückzuhalten.

6. Verfahren gemäß einem der Ansprüche 2 bis 4, wobei der Feststoff, der in Schritt c) erhalten wird, ohne Waschen gefiltert und gepresst wird, um so viel Zuckerlösung wie möglich vor Schritt d) zu entfernen.

7. Verfahren gemäß einem der Ansprüche 1 bis 6, wobei das Gemisch in Schritt (c) für eine lange Kontaktzeit, z. B. Stunden, Tage, Wochen oder Monate, gehalten wird, um Proteinbindung zu ermöglichen und/oder um Keimung von Pollen, die in dem Honig vorhanden sind, zu ermöglichen.

8. Verfahren nach einem der Ansprüche 1 bis 7, wobei das Harz von der Flüssigphase durch Zentrifugation getrennt wird.

9. Verfahren nach einem der Ansprüche 1 bis 8, wobei das Kationenaustauschmedium SP-Cellulose-HG2-Harz ist.

10. Bienenproduktextrakt, der durch das Verfahren nach einem der Ansprüche 1 bis 9 erhalten wird oder erhaltbar ist.

11. Pharmazeutische Zusammensetzung, umfassend einen Bienenproduktextrakt gemäß Anspruch 10.

12. Pharmazeutische Zusammensetzung gemäß Anspruch 11, wobei die Zusammensetzung als eine topische oder sublinguale Zubereitung formuliert ist.

## Revendications

1. Procédé par lots de traitement du miel de manuka pour former un extrait de produit des abeilles, le procédé comprenant :
a) la combinaison du miel avec de l'eau et une résine échangeuse de cations comprenant un ligand soufre propyle ou p-toluènesulfonyle et une matrice de cellulose ou d'agarose ;
b) l'ajustement du pH du mélange à un pH compris dans la plage de 2,5 à 3,0 ;
c) la filtration du mélange pour former un solide, et éventuellement le séchage du solide ;
d) le mélange du solide avec de l'eau ;
e) l'ajustement du pH du mélange entre 8,0 et 10,5 ;
f) la filtration du mélange et la récupération du filtrat ; et
g) éventuellement, la répétition des étapes b) à f).

2. Procédé selon la revendication 1, dans lequel la résine échangeuse de cations est un matériau polymère SP sous forme solide.

3. Procédé selon la revendication 2, dans lequel la résine est une résine SP à base d'agarose telle que SP Big Beads, ou SP cellulose d'une taille de particule suffisante pour permettre la filtration et des procédés basés sur la pression pour récupérer le filtrat à partir du solide.

4. Procédé selon la revendication 2 ou 3, dans lequel les étapes de filtration c) et f) sont effectuées par un filtre en tissu, un filtre-presse ou une filtration RVF, de préférence par un filtre-presse.

5. Procédé selon l'une quelconque des revendications 2 à 4, dans lequel le solide obtenu à l'étape c) est lavé avec de l'eau à un pH bas pour diminuer la teneur en sucre et retenir la protéine liée à la résine.

6. Procédé selon l'une quelconque des revendications 2 à 4, dans lequel le solide obtenu à l'étape c) est filtré et pressé sans lavage, pour éliminer autant de solution de sucre que possible avant l'étape d).

7. Procédé selon l'une quelconque des revendications 1 à 6, dans lequel le mélange est maintenu à l'étape (c) pendant une longue durée de contact, par exemple des heures, des jours, des semaines ou des mois, pour permettre la liaison aux protéines et/ou pour permettre la germination du pollen présent dans le miel.

8. Procédé selon l'une quelconque des revendications 1 à 7, dans lequel la résine est séparée de la phase liquide par centrifugation.

9. Procédé selon l'une quelconque des revendications 1 à 8, dans lequel le milieu échangeur de cations est une résine SP cellulose HG2.

10. Extrait de produit des abeilles obtenu ou pouvant être obtenu par le procédé selon l'une quelconque des revendications 1 à 9.

11. Composition pharmaceutique comprenant un extrait de produit des abeilles selon la revendication 10.

12. Composition pharmaceutique selon la revendication 11, dans laquelle la composition est formulée sous la forme d'une préparation topique ou sublinguale.
